# EUROPEAN PATENT APPLICATION

(11) **EP 4 089 103 A1**
(43) Date of publication of application: **16.11.2022**
(21) Application number: 21173549.3
(22) Date of filing: 12.05.2021
(51) Int. Cl.: C07K 7/64, A61P 31/06, G01N 33/68, A61K 38/00

(54) **CLPCP PROTEASE-BINDING CYCLIC HEPTAPEPTIDES**

(71) Applicant: Universität Duisburg-Essen, 45141 Essen (DE); Boehringer Ingelheim International GmbH, 55216 Ingelheim am Rhein (DE)
(72) Inventor: Meinhart, Anton, 1030 Vienna (AT); Clausen, Tim, 1030 Vienna (AT); Morreale, Francesca, 1030 Vienna (AT); Leodolter, Julia, 1030 Vienna (AT); Kleine, Stefan, 45417 Essen (DE); Kaiser, Markus, 46238 Bottrop (DE)
(74) Representative: Michalski Hüttermann & Partner Patentanwälte mbB

(57) **Abstract**

The present invention relates to compounds being or comprising a ClpCP protease-binding cyclic heptapeptide that are usable in the treatment of mycobacterial infections. The invention further relates to screening methods for compounds that act as protein degradation inducers (PROTACs) or for target proteins for induced protein degradation using the compounds, as well as to a pharmaceutical composition comprising the compounds.

## Description

The present invention relates to compounds being or comprising a ClpCP protease-binding cyclic heptapeptide.

The ClpC:ClpP (ClpCP) protease is a central quality-control factor in Gram-positive bacteria. This proteolytic complex is found in Gram-positive bacteria, such as *Bacillus subtilis (B. subtilis)* and *Staphylococcus aureus (S. aureus),* and mycobacteria, such as *Mycobacterium tuberculosis (M. tuberculosis),* where the equivalent ClpC1:ClpP1P2 (ClpC1P1P2) protease is essential for survival *in vitro* and in macrophages. The hexameric AAA (ATPases Associated with diverse cellular Activities) protease ClpCP constitutes the core of the protein quality control system in Gram-positive bacteria, removing damaged proteins during stress situations. Proteins that need to be degraded are labeled by a pArg degradation mark and thus recognized by the N-terminal domain (NTD) of the ClpC unfoldase, which then translocates the captured client protein into the proteolytic cage formed by ClpP. This pArg-ClpCP degradation system represents a simple, bacterial version of the eukaryotic ubiquitinproteasome pathway. Given their crucial role in ClpC / ClpC1 function regulation and substrate recognition, NTDs are also the target of antimycobacterial cyclic peptides Cyclomarin A (CymA), ecumicin and lassomycin.

The possibility of hijacking cellular protein-tagging and degradation machineries offers unique opportunities for drug discovery, as demonstrated by the development of proteolysis-targeting chimeras (PROTACs). WO 2013/106643 A1 discloses bifunctional compounds comprising a ubiquitin ligase binding moiety that is connected via a linker group to a protein target moiety which binds to a target protein or polypeptide which is to be ubiquitinated by the ubiquitin ligase placed in proximity to the target protein via such bifunctional compound. Once tagged with multiple ubiquitin molecules, the target protein is subsequently degraded via the eukaryotic proteasome protein degradation pathway.

The ClpC:ClpP (ClpCP) protease holds great potential for developing a bacterial PROTAC system that would allow for targeted, small molecule-induced protein degradation in bacteria. This system for example may be usable as an alternative chemotherapeutic strategy against pathogenic bacteria, which still represent a major threat to human health.

The naturally occurring antibacterial cyclic peptide Cyclomarin A (CymA) kills *Mycobacterium tuberculosis* cells by binding to the NTD of ClpC overruling ClpC activity control and causing persistent activation. *Mycobacterium tuberculosis* still is one of the most threatening bacterial pathogens worldwide, and tuberculosis continues to be a devastating human disease despite many efforts in pharmaceutical treatment.

Therefore, the object underlying the present invention was to provide compounds that provide antibacterial properties or are able to reprogram the bacterial degradation machines to combat microbial infections.

The problem is solved by the compound according to claim 1. The problem further is solved by the compounds for use according to claim 8, a pharmaceutical composition according to claim 9 and the methods of claims 10 to 13 and 15, and a use of the compounds according to claim 14. Preferred embodiments of the invention are given by the dependent claims, which can constitute each solely or in combination an aspect of the invention.

According to the invention is provided a compound according to general formula (1) or a pharmaceutically acceptable salt, stereoisomer, solvate or polymorph thereof: 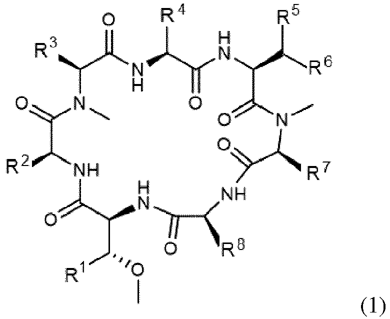 wherein:
- R¹: is a 5- or 6-membered aromatic or heteroaromatic ring or a 9- or 10-membered aromatic or heteroaromatic ring system, optionally substituted with one or more groups independently selected from NO₂, CN, OH, halogen, optionally substituted C₁-C₆ alkyl, optionally substituted C₂-C₆ alkenyl, or optionally substituted C₁-C₆ alkoxy where the substitution may be NO₂, CN, OH, COOH, or halogen;
- R², R³, R⁴: are each independently selected from the group comprising H, NO₂, CN or halogen optionally substituted C₁-C₆ alkyl, optionally substituted C₂-C₆ alkenyl, optionally substituted C₁-C₆ alkoxy, optionally substituted -(CH₂)ₘ-S-(CH₂)ₘ-CH₃, optionally substituted -(CH₂)ₘ-O-(C₁-C₄) alkyl, optionally substituted -(CH₂)ₘ-OH, optionally substituted -(CH₂)ₘ-CH(OH)-(CH₂)ₘ-CH₃, optionally substituted -(CH₂)ₘ-SH, optionally substituted -(CH₂)ₘ-NR^{1N}R^{2N}, optionally substituted -(CH₂)ₘ-C(O)-NR^{1N}R^{2N}, optionally substituted -(CH₂)ₘ-NH-C(NR^{1N}R^{2N})NR^{1N}, optionally substituted -(CH₂)ₘ-NHC(O)R^{1N}, optionally substituted -(CH₂)ₘ-COOH, where the substitution may be C₁-C₃ alkyl, C₁-C₃ alkoxy, NO₂, CN, OH, COOH, or halogen;
- R⁵: is a 5- or 6-membered aromatic or heteroaromatic ring or a 9- or 10-membered aromatic or heteroaromatic ring system, optionally substituted with one or more groups independently selected from NO₂, CN, OH, COOH, halogen, or optionally substituted C₁-C₆ alkyl, optionally substituted C₂-C₆ alkenyl, optionally substituted C₁-C₆ alkoxy where the substitution may be NO₂, CN, OH, COOH, or halogen;
- R⁶: is selected from the group comprising H and OH;
- R⁷: is -(CH₂)ₙ-NR^{3N}R^{4N};
- R⁸: is selected from R² or is a moiety bound to a linker which covalently links the moiety to a targeting group, and the targeting group is capable of binding to a target protein or (poly)peptide which is to be degraded by ClpCP protease;
- R^{1N}R^{2N}: are each independently selected from the group comprising H and C₁-C₃ alkyl;
- R^{3N}, R^{4N}: are each independently selected from the group comprising H, OH, COOH, CO, -(CH₂)-OH and C₁-C₃ alkyl;
- m: is each independently 0, 1, 2, 3, 4, 5 or 6;
- n: is 1, 2, 3, 4, 5 or 6.

The compound in embodiments provides a ClpCP protease-binding synthetic cyclic heptapeptide (in the following also denoted "sCymA") or is a ClpCP protease-binding synthetic cyclic heptapeptide bound to a linker which covalently links the heptapeptide to a targeting group that is capable of binding to a target protein which is to be degraded by ClpCP protease (in the following also denoted "sCymA-BacPROTAC" or "bacPROTAC").

The synthetic cyclic heptapeptide comprises amino acids which are simple or standard amino acids compared to the naturally occurring antibacterial cyclic peptide Cyclomarin A (CymA). The simplified Cyclomarin A mimetics are in the following also denoted "sCymA". Surprisingly it was found that the structurally simplified synthetic cyclic heptapeptide bind to the substrate-receptor domain of the ClpC:ClpP protease and is able to exhibit antibacterial properties *in vitro.* Advantageously, the simplified synthetic cyclic heptapeptides exhibit similar functional properties as the complex natural peptide and can enter the mycobacterial cell and induce cell death. It could be shown that the compounds can provide minimum inhibitory concentrations (MIC) in the low micromolar range. Further, these simplified cyclic heptapeptides are composed of less complex chemical building blocks and a reduced number of unconvential amino acids and can be synthesized by fast and efficient synthesis routines.

The sCymA compounds can be connected to a second functional group and thus can bring a target protein and the ClpCP protease into proximity. When linked to a targeting group that is capable of binding a protein of interest (i.e. the target protein), the cyclic heptapeptide binds to ClpC:ClpP protease, brings the protein of interest (target protein) into proximity of the protease and thus enables its ClpC-mediated protein degradation in bacteria. In the following, these compounds which are able to direct proteins of interest to ClpCP-mediated degradation are denoted bacterial PROTACs or "bacPROTACs". Experimental results show that the bacPROTACs can mediate protein degradation by ClpCP and ClpC1P₁P₂ *in vitro.* Degradation assays performed in mycobacteria further demonstrate activity *in vivo.* This shows that the bacPROTACs can induce selected protein degradation *in vitro* and *in vivo* and provide a modular system to target mycobacterial proteins of interest for degradation. In summary, experimental results show that the compounds of the invention and particularly the bacPROTACs provide the potential of next generation antibiotics. In this respect, it is highly advantageous that the targeting groups (binders) do not have to compromise ClpC function or function of other target proteins to provide a pharmaceutical effect, as known antibiotics do. This is a general advantage of the compounds over small molecule inhibitors. Moreover, such bacPROTACs may not be degraded themselves in such degradation cycle, but may recycle and bind to a further target protein molecule, initiating the next degradation cycle, thereby acting in a "catalytic" manner, as compared to the "stoichiometric" mode of "classical" small molecule protein modulators.

The term "alkyl" according to the invention is to be understood as meaning straight-chain or branched alkyl groups. The term "C₁-C₆-alkyl" as used herein refers to alkyl groups having 1 to 6 carbon atoms. C₁-C₆-Alkyl groups may be selected from the group comprising methyl, ethyl, propyl, butyl, pentyl and hexyl. Preferred are C₁-C₃-alkyl groups, particularly methyl and ethyl.

The term "alkenyl" is to be understood as meaning straight-chain or branched alkyl groups having at least one double bond between carbon atoms.

The term "C₁-C₆-alkoxy" is to be understood as meaning a C₁-C₆-alkyl bonded to oxygen. Preferred are C₁-C₃-alkoxy groups, particularly methoxy and ethoxy.

The term "heteroaromatic" is to be understood as meaning an aromatic ring or ring system comprising at least one hetero atom selected from O, S and N.

The term "ring system" particularly includes fused systems of aromatic rings such as indole.

The term "halogen" according to the invention is to be understood as meaning fluorine, chlorine, bromine or iodine, preferably fluorine, chlorine or bromine.

In embodiments, the groups R¹ to R⁸ provide side chains of the cyclic heptapeptide, which may be side chains of naturally occurring amino acids or may be side chains that are not naturally occuring such as of synthetic amino acids.

The group R¹ is an optionally substituted 5- or 6-membered aromatic or heteroaromatic ring or 9- or 10-membered aromatic or heteroaromatic ring system. Also the group R⁵ is an optionally substituted 5- or 6-membered aromatic or heteroaromatic ring or 9- or 10-membered aromatic or heteroaromatic ring system. Preferred substituents are independently selected from OH, COOH, C₁-C₆ alkoxy, or optionally substituted C₁-C₆ alkyl, where the substitution may be NO₂, CN, OH, or COOH. The optionally substituted 5- or 6-membered aromatic or heteroaromatic ring or 9- or 10-membered aromatic or heteroaromatic ring system may be selected from the group comprising phenyl, phenol, imidazole, indole, furan, thiophene, pyrrole, pyrrolidine, piperidine, piperazine, pyridine, pyrimidine and morpholine.

In embodiments of the compound, R¹ and R⁵ are independent from each other selected from the group comprising phenyl, phenol, imidazole and indole, optionally substituted with one or more groups selected from C₁-C₆ alkyl, C₂-C₆ alkenyl, C₁-C₆ alkoxy, NO₂, CN, OH, COOH, or halogen. In embodiments R¹ is selected from phenyl and phenol. In embodiments R¹ is phenyl. In preferred embodiments, R⁵ is selected from imidazole and indole. In preferred embodiments, R⁵ is indole.

The group R⁶ may be selected from H and OH. In embodiments, R⁶ may be hydroxyl. Such embodiments share the hydroxyl group with the cyclic peptide Cyclomarin A (CymA). In preferred embodiments, R⁶ may be hydrogen. Such embodiments are easy to synthesize.

In embodiments of the compound, R², R³, R⁴ may be each independently selected from the group comprising H, optionally substituted C₁-C₆ alkyl, optionally substituted C₂-C₆ alkenyl, optionally substituted C₁-C₆ alkoxy, optionally substituted -(CH₂)ₘ-S-(CH₂)ₘ-CH₃, optionally substituted - (CH₂)ₘ-O-(C₁-C₄) alkyl, optionally substituted -(CH₂)ₘ-OH, optionally substituted -(CH₂)ₘ-NR^{1N}R^{2N}, optionally substituted -(CH₂)ₘ-COOH, where the substitution may be C₁-C₃ alkyl, C₁-C₃ alkoxy, NO₂, CN, OH, COOH, or halogen; R^{1N}R^{2N} are each independently selected from the group comprising H and C₁-C₃ alkyl; and m is each independently 0, 1, 2, 3, 4, 5 or 6.

In embodiments, R², R³, R⁴ may be each independently selected from the group comprising comprising hydrogen, optionally substituted C₁-C₆ alkyl, optionally substituted C₂-C₆ alkenyl, optionally substituted C₁-C₆ alkoxy, optionally substituted -(CH₂)ₘ-S-(CH₂)ₘ-CH₃, optionally substituted -(CH₂)ₘ-O-(C₁-C₃) alkyl, wherein the substitution may be NO₂, C₁-C₃ alkoxy CN, OH, or halogen, and m is each independently 0, 1, 2 or 3. In embodiments, R², R³, R⁴ may be each independently selected from the group comprising hydrogen, -CH(CH₃)₂, -CH₂-CH(CH₃)₂, - CH(CH₃)CH₂-CH₃ and -(CH₂)₂-S-CH₃.

In embodiments, R⁷ is -(CH₂)ₙ-NR^{3N}R^{4N}; where R^{3N}, R^{4N} are each independently selected from the group comprising H, OH, COOH, CO, -(CH₂)-OH and C₁-C₃ alkyl; and n is 1, 2, 3, 4, 5 or 6.

In embodiments, the group R⁸ is selected from R². In such embodiments the compound provides a ClpCP protease-binding synthetic cyclic heptapeptide (sCymA). Such a compound can provide antibacterial effects. In embodiments of the compound being a ClpCP protease-binding synthetic cyclic heptapeptide or antibiotic, R⁸ may be -CH₃.

In other embodiments, the group R⁸ is a moiety bound to a linker which covalently links the moiety to a targeting group, while the targeting group is capable of binding to a target protein or polypeptide which is to be degraded by ClpCP protease. In these embodiments, the moiety may be or R⁸ may comprise a side chain of an amino acid that is covalently bonded via a COOH or NH₂ end group to the linker. In these embodiments the compound comprises a ClpCP protease-binding heptapeptide and a linker. In such embodiments the compound provides a "proteolysis targeting chimera" (PROTAC) designed to bind with one part of the compound, i.e. the target moiety, to a target protein and with the other part, i.e. the synthetic cyclic heptapeptide, to ClpCP protease, which complex is designed to result in the degradation of the target protein by ClpCP protease. The compound of those embodiments may be referred to as "sCymA-bacPROTAC".

In embodiments, the compound is a compound according to general formula (2) or a pharmaceutically acceptable salt, stereoisomer, solvate or polymorph thereof: 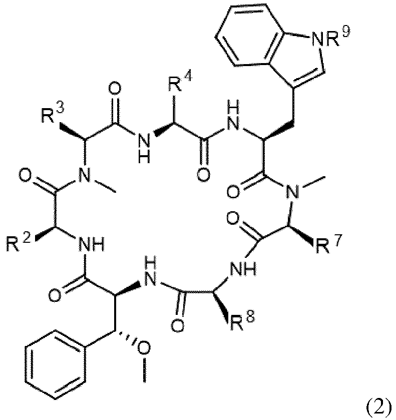 wherein:
- R², R³, R⁴: are each independently selected from the group comprising H, optionally substituted C₁-C₆ alkyl, optionally substituted C₂-C₆ alkenyl, optionally substituted C₁-C₆ alkoxy, optionally substituted -(CH₂)ₘ-S-(CH₂)ₘ-CH₃, optionally substituted -(CH₂)ₘ-O-(C₁-C₃) alkyl, wherein the substitution may be NO₂, C₁-C₃ alkoxy CN, OH, or halogen,
- R⁷: is -(CH₂)ₙ-NR^{3N}R^{4N};
- R⁹: is selected from the group comprising H, C₁-C₆ alkyl, C₂-C₆ alkenyl, or C₁-C₆ alkoxy,
- R^{3N}, R^{4N}: are each independently selected from the group comprising H, OH, CO,-(CH₂)-OH and C₁-C₃ alkyl;
- m: is each independently 0, 1, 2 or 3;
- n: is 1, 2, 3, 4, 5 or 6; and
- R⁸: is selected from R² or is a moiety bound to a linker which covalently links the moiety to a targeting group, and the targeting group is capable of binding to a target protein or (poly)peptide which is to be degraded by ClpCP protease.

In embodiments of the compound are:
- R², R³, R⁴: each independently selected from the group comprising H, -CH(CH₃)₂, -CH₂-CH(CH₃)₂, -CH(CH₃)CH₂-CH₃ and -(CH₂)₂-S-CH_{3;}
- R⁷: -(CH₂)ₙ-NR^{3N}R^{4N} wherein R^{3N}, R^{4N} are each independently selected from the group comprising H, OH, CO,-(CH₂)-OH and C₁-C₃ alkyl, and n is 1, 2, 3, 4, 5 or 6,
- R⁸: selected from R² or is a moiety bound to a linker which covalently links the moiety to a targeting group, and the targeting group is capable of binding to a target protein or (poly)peptide which is to be degraded by ClpCP protease, and
- R⁹: selected from the group comprising H, C₁-C₆ alkyl, C₂-C₆ alkenyl and C₁-C₆ alkoxy.

In embodiments of the compound, R² is -CH(CH₃)₂; R³ is -CH₂-CH(CH₃)₂; R⁴ is -CH(CH₃)CH₂-CH₃; R⁷ is -(CH₂)ₙ-NR^{3N}R^{4N} wherein R^{3N}, R^{4N} are each independently selected from the group comprising H, OH, CO, -(CH₂)-OH and C₁-C₃ alkyl, and n is 1, 2, 3, 4, 5 or 6; and R⁹ is hydrogen.

In embodiments, R², R³, R⁴ provide groups referring to side chains of small nonpolar amino acids selected from the group of glycine, valine, leucine, isoleucine and methionine.

In embodiments, the compound can comprise modified amino acids phenylalanine, valine, leucine, isoleucine, tryptophan, lysine and alanine, according to the following formula (4)

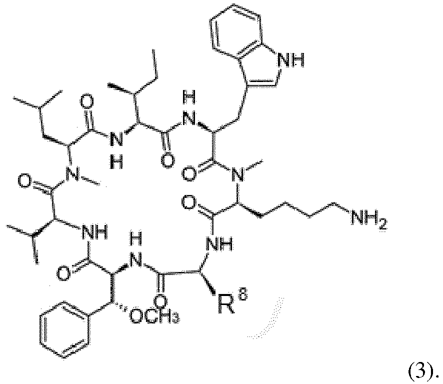

In embodiments where the compound provides a bacterial "proteolysis targeting chimera" (bacPROTAC) designed to bind with the cyclic heptapeptide part of the compound to ClpCP protease and via the targeting group to a target molecule, R⁸ is a moiety comprising a linker and a targeting moiety. In preferred embodiments of the compound, R⁸ is a moiety, such as an amino acid side chain, bound to a linker which covalently links the moiety to a targeting group, and the targeting group is capable of binding to a target protein or (poly)peptide which is to be degraded by ClpCP protease. In these embodiments, the compound may have the formula (4): 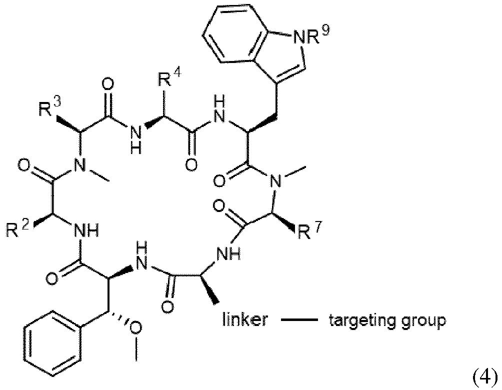 where the linker is a group which covalently links the ClpCP protease-binding synthetic cyclic heptapeptide to the targeting moiety; and the targeting group is a chemical moiety which binds to a target protein or (poly)peptide which is to be degraded by ClpCP protease and is covalently linked to the linker.

The linker may be bound via an amide group to the side chain of an amino acid comprising an amine or carboxyl group, such as lysine, glutamic acid or aspartic acid. In embodiments, R⁸ further comprises or the R⁸ moiety is the remains of the respective side chain, such as an -(CH₂)ₙ-group where n is 1, 2 or 4 in case of aspartic acid, glutamic acid or lysine being the respective amino acid of the heptapeptide.

In embodiments, the linker is selected from polyethylene glycole, a peptide linker, an alkyl linker and/or -((CH₂)ₓN(CH₃))ₙ- wherein x is 1 or 2 and n is an integer in the range of 1 to 24. Preferably, a polyethylene glycol (PEG) group comprises 1 to 24, 2 to 12 or 3 to 5 polyethylene glycol units. Preferred are linear polyethylene glycol units -(CH₂CH₂O)ₙ-, wherein n is an integer in a range of 1 to 24, in a range of 12 to 24, or in a range of 2 to 12, preferably of 3 to 5. In other embodiments the linker is a peptide linker, wherein the peptide linker may comprise amino acids selected from the group comprising glycine and/or alanine. A peptide linker may comprise 1 to 24, preferred of 2 to 12, more preferred 2 to 4 amino acids. In other embodiments the linker may be a linear or branched, saturated or unsaturated C₁-C₄₀-alkyl group, preferred a C₈-C₂₀ alkyl group, more preferred a C1₂-C₂₀-alkyl group. The linker may be an alkyl group -(CH₂)ₙ- wherein n is an integer in the range of 1 to 40, in the range of 8 to 20, or in the range of 12 to 20.

Different targeting groups that are capable of binding to many different target proteins or polypeptides which are to be degraded by ClpCP protease can be linked to the heptapeptide. The modularity of the compounds (bacPROTACs) allows testing the degradation of many different target proteins. Currently, over 90000 compounds targeting 73 different *Mycobacterium tuberculosis* (Mtb) targets are reported in the CHEMBL database. Each of those 90000 compounds thus provides a usable targeting group for targeting *Mycobacterium tuberculosis* proteins.

In embodiments, the targeting group is selected from the group comprising biotin, SLF' (synthetic ligand of FKBP', also denoted 1-(3,3-dimethyl-1,2-dioxopentyl)-(2S)-2-piperidinecarboxylic acid, (1R)-1-(3-aminophenyl)-3-(3,4-dimethoxyphenyl)propyl ester), trimethoprim (TMP) and JQ1. JQ1 is a thienotriazolodiazepine and a small-molecule inhibitor of bromodomain proteins, binding to all bromodomains of the Bromodomain and Extra-terminal motif (BET) protein family. TMP binds and inhibits of dihydrofolate reductase (dfrA, alternatively named folA or dhfr), an essential gene in Mtb. JQ1, for example, as the targeting group, is usable in the experimental setting as disclosed herein, for targeting *Mycobacterium tuberculosis* (Mtb) target proteins or proteins of other Gram-positive bacteria, by using fusion proteins consisting of such target protein fused to a Bromodomain testisspecific (BRDT) protein. Specifically, bacterial cells can be modified by fusing the BRDT protein sequence to one of the 73 *Mycobacterium tuberculosis* (Mtb) target proteins or any other target protein of Gram-positive bacteria and allowing BRDT to be expressed with the bacterial protein, so that the compound (bacPROTACs) comprising JQ1 as the targeting group will bind via BRDT to any bacterial target protein and induce its degradation.

Proteins, particularly essential proteins, of mycobacteria tuberculous species and strains belonging to the Mycobacterium tuberculosis complex (MTBC), of non-tuberculous species and strains belonging to the Mycobacterium avium complex (MAC) except the non-pathogenic *Mycobacterium indicus pranii,* and other important pathogenic mycobacteria such as species belonging to the clades of *Mycobacterium kansasii, Mycobacterium abscessus*/*chelonae* or *Mycobacterium fortuitum; Mycobacterium ulcerans, Mycobacterium leprae, Mycobacterium lepromatosis* and *Mycobacterium lepraemurim* or proteins of other Gram-positive bacteria are usable target proteins. Mycobacteria tuberculous species and strains belonging to the Mycobacterium tuberculosis complex (MTBC) are *e.g. Mycobacterium tuberculosis, Mycobacterium africanum, Mycobacterium bovis, (including Bacillus Calmette-Guérin strain), Mycobacterium canetti, Mycobacterium caprae, Mycobacterium microti, Mycobacterium orygis, Mycobacterium tuberculosis* subsp. *caprae, Mycobacterium pinnipedii, Mycobacterium suricattae, Mycobacterium mungi.* Non-tuberculous species and strains belonging to the *Mycobacterium avium* complex (MAC) except the non-pathogenic *Mycobacterium indicus pranii,* are e.g. *Mycobacterium avium subsp. avium, Mycobacterium. avium subsp. paratuberculosis, Mycobacterium avium subsp. silvaticum, Mycobacterium avium subspecies "hominissuis*" *Mycobacterium colombiense, Mycobacterium intracellulare, Mycobacterium yongonense.*

Preferred are *Mycobacterium tuberculosis* proteins selected from the following table 1:

**Table 1: Mycobacterium tuberculosis proteins usable as target proteins**

| Protein name (according to ChEMBL) | Uniprot Entry | M. tub. H37Rv locus (according to Mycobrowser) |
|---|---|---|
| *Thymidylate synthase thyX* | P9WG57 | Rv2754c |
| *ATP phosphoribosyltransferase* | P9WMN1 | Rv2121c |
| *3-dehydroquinate dehydratase* | P9WPX7 | Rv2537c |
| *Alanine racemase* | P9WQA9 | Rv3423c |
| *Inorganic polyphosphate*/*ATP-NAD kinase* | P9WHV7 | Rv1695 |
| *1,4-Dihydroxy-2-naphthoyl-CoA synthase* | P9WNP5 | Rv0548c |
| *dTDP-4-dehydrorhamnose reductase* | P9WH09 | Rv3266c |
| *Thymidylate kinase* | P9WKE1 | Rv3247c |
| *6,7-dimethyl-8-ribityllumazine synthase* | P9WHE9 | Rv1416 |
| *1-deoxy-D-xylulose-5-phosphate synthase* | P9WNS3 | Rv2682c |
| *1-deoxy-D-xylulose 5-phosphate reductoisomerase* | P9WNS1 | Rv2870c |
| *Thioredoxin reductase* | P9WHH1 | Rv3913 |
| *Fructose-bisphosphate aldolase* | P9WQA3 | Rv0363c |
| *Ribose-5-phosphate isomerase B* | P9WKD7 | Rv2465c |
| *Dihydrodipicolinate synthase* | P9WP25 | Rv2753c |
| *Pantothenate synthetase* | P9WIL5 | Rv3602c |
| *Phospho-N-acetylmuramoyl-pentapeptide-transferase* | P9WMW7 | Rv2156c |
| *Arabinosyltransferase A* | P9WNL9 | Rv3794 |
| *D-alanylalanine synthetase* | P9WP31 | Rv2981c |
| *UDP-galactopyranose mutase* | P9WIQ1 | Rv3809c |
| *Cell division protein FtsZ* | P9WN95 | Rv2150c |
| *Bifunctional protein glmU* | P9WMN3 | Rv1018c |
| *UDP-galactofuranosyl transferase GlfT2* | O53585 | Rv3808c |
| *DNA-directed RNA polymerase beta chain* | P9WGY9 | Rv0667 |
| *Peptide deformylase* | P9WIJ3 | Rv0429c |
| *DNA ligase* | P9WNV1 | Rv3014c |
| *Ribonucleoside-diphosphate reductase subunit alpha* | P9WH75 | Rv3051c |
| *Replicative DNA helicase* | P9WMR3 | Rv0058 |
| *DNA gyrase subunit A* | P9WG47 | Rv0006 |
| *3-oxoacyl-[acyl-carrier-protein] synthase 1* | P9WQD9 | Rv2245 |
| *Fatty acid synthase* | P95029 | Rv2524c |
| *Enoyl-[acyl-carrier-protein] reductase [NADH] (InhA)* | M9TGV3 | Rv1484 |
| *Serine*/*threonine-protein kinase pknB* | P9WI81 | Rv0014c |
| *L-cysteine:1D-myo-inositol 2-amino-2-deoxy-alpha-D-glucopyranoside ligase* | P9WJM9 | Rv2130c |
| *Adenosylmethionine-8-amino-7-oxononanoate aminotransferase* | P9WQ81 | Rv1568 |
| *Dihydrofolate reductase* | P9WNX1 | Rv2763c |
| *Thymidylate synthase (ThyA)* | P9WFR9 | Rv2764c |
| *Uncharacterized protein Rv1284*/*MT1322* | P9WPJ7 | Rv1284 |
| *Putative cytochrome P450 125* | P9WPP1 | Rv3545c |
| *Isocitrate lyase* | P9WKK7 | Rv0467 |
| *Carbonic anhydrase* | P9WPJ9 | Rv3588c |
| *3-oxoacyl-[acyl-carrier-protein] synthase 2* | P9WQD7 | Rv2246 |
| *Probable serine*/*threonine-protein kinase pknG* | P9WI73 | Rv0410c |
| *Phosphotyrosine protein phosphatase* | A0A045ISB3 | Rv0153c |
| *Intracellular chorismate mutase* | P9WIC1 | Rv0948c |
| *LmbE-related protein* | P9WJN3 | Rv1170 |
| *Glutathione reductase homolog* | P9WHH3 | Rv2855 |
| *HTH-type transcriptional regulator EthR* | P9WMC1 | Rv3855 |
| *Epoxide hydrolase* | P95276 | Rv1938 |
| *3 beta-hydroxysteroid dehydrogenase*/*Delta 5-- >4-isomerase* | P9WQP7 | Rv1106c |
| *PROBABLE TRANSMEMBRANE CARBONIC ANHYDRASE (CARBONATE DEHYDRATASE) (CARBONIC DEHYDRATASE)* | P96878 | Rv3273 |
| *O-acetylserine sulfhydrylase* | P9WP55 | Rv2334 |
| *PYRAZINAMIDASE*/*NICOTINAMIDAS PNCA (PZase)* | Q50575 | Rv2043 |
| *Chorismate mutase-related protein* | P9WIB9 | Rv1885c |
| *Purine nucleoside phosphorylase* | P9WP01 | Rv3307 |
| *Putative cytochrome P450 130* | P9WPN5 | Rv1256c |
| *Lanosterol 14-alpha demethylase* | P9WPP9 | Rv0764c |
| *Probable L-lysine-epsilon aminotransferase* | P9WQ77 | Rv3290c |
| *Cytochrome P450 121* | P9WPP7 | Rv2276 |
| *Putative uncharacterized protein* / *Deazaflavin-dependent nitroreductase* | P9WP15 | Rv3547 |
| *Acyl-CoA synthase* | P9WQ59 | Rv2941 |
| *3-oxoacyl-[acyl-carrier-protein] synthase III* | P9WNG3 | Rv0533c |
| *Fibonectin-binding protein C* | P9WQN9 | Rv0129c |
| *POSSIBLE FATTY-ACID-CoA SYNTHETASE FADD17 (FATTY-ACID-CoA SYNTHASE) (FATTY-ACID-CoA LIGASE)* | 053551 | Rv3506 |
| *2,3-dihydroxybenzoate-AMP ligase* | P71716 | Rv2384 |
| *PROBABLE FATTY-ACID-CoA LIGASE FADD19 (FATTY-ACID-CoA SYNTHETASE) (FATTY-ACID-CoA SYNTHASE)* | P9WQ51 | Rv3515c |
| *Serine*/*threonine-protein kinase pknF* | P9WI75 | Rv1746 |
| *Transcriptional regulatory protein devR (dosR)* | P9WMF9 | Rv3133c |
| *Probable low molecular weight protein-tyrosine-phosphatase* | P9WIA1 | Rv2234 |
| *Protein RecA* | P9WHJ3 | Rv2737c |
| *Mycothiol S-conjugate amidase* | P9WJN1 | Rv1082 |
| *ATP synthase gamma chain* | P9WPU9 | Rv1309 |
| *ATP synthase subunit a* | P9WPV7 | Rv1304 |
| *ATP synthase subunit b-delta* | P9WPV3 | Rv1307 |
| *ATP synthase subunit b* | P9WPV5 | Rvl306 |
| *ATP synthase subunit beta* | P9WPU5 | Rv1310 |
| *ATP synthase subunit c* | P9WPS 1 | Rv1305 |
| *ATP synthase subunit alpha* | P9WPU7 | Rvl308 |
| *ATP synthase epsilon chain* | P9WPV1 | Rv1311 |

The target protein may be a single protein, possibly of a larger multiprotein complex, e.g. essential for bacterial intermediary metabolism and respiration, cell wall and cell processes, information pathways, lipid metabolism or regulatory proteins. The target protein, for example, may be selected from the group of Thymidylate synthase thyX, ATP phosphoribosyltransferase, 3-dehydroquinate dehydratase, Alanine racemase, Inorganic polyphosphate/ATP-NAD kinase, 1,4-Dihydroxy-2-naphthoyl-CoA synthase, dTDP-4-dehydrorhamnose reductase, Thymidylate kinase, 6,7-dimethyl-8-ribityllumazine synthase, 1-deoxy-D-xylulose-5-phosphate synthase, 1-deoxy-D-xylulose 5-phosphate reductoisomerase, Thioredoxin reductase, Fructose-bisphosphate aldolase, Ribose-5-phosphate isomerase B, Dihydrodipicolinate synthase, Pantothenate synthetase, Phospho-N-acetylmuramoyl-pentapeptide-transferase, Arabinosyltransferase A, D-alanylalanine synthetase UDP-galactopyranose mutase, Cell division protein FtsZ, Bifunctional protein glmU, UDP-galactofuranosyl transferase GlfT2, DNA-directed RNA polymerase beta chain, Peptide deformylase, DNA ligase, Ribonucleoside-diphosphate reductase subunit alpha, Replicative DNA helicase, DNA gyrase subunit A, 3-oxoacyl-[acyl-carrier-protein] synthase 1, Fatty acid synthase, Enoyl-[acyl-carrier-protein] reductase [NADH], Serine/threonine-protein kinase pknB, L-cysteine:1D-myo-inositol 2-amino-2-deoxy-alpha-D-glucopyranoside ligase, Adenosylmethionine-8-amino-7-oxononanoate aminotransferase, Dihydrofolate reductase, Thymidylate synthase, Uncharacterized protein Rv1284/MT1322, Putative cytochrome P450 125, Isocitrate lyase, Carbonic anhydrase, 3-oxoacyl-[acyl-carrier-protein] synthase 2, Probable serine/threonine-protein kinase pknG, Phosphotyrosine protein phosphatase, Intracellular chorismate mutase, LmbE-related protein, Glutathione reductase homolog, HTH-type transcriptional regulator EthR, Epoxide hydrolase, 3 beta-hydroxysteroid dehydrogenase/Delta 5-->4-isomerase, Probable transmembrane carbonic anhydrase (carbonate dehydratase) (carbonic dehydratase), O-acetylserine sulfhydrylase, pyrazinamidase/nicotinamidas PNCA (PZase), Chorismate mutase-related protein, Purine nucleoside phosphorylase, Putative cytochrome P450 130, Lanosterol 14-alpha demethylase, Probable L-lysine-epsilon aminotransferase, Cytochrome P450 121, Putative uncharacterized protein / Deazaflavin-dependent nitroreductase, Acyl-CoA synthase, 3-oxoacyl-[acyl-carrier-protein] synthase III, Fibonectin-binding protein C, Possible fatty-acid-CoA synthetase FADD17 (fatty-acid-CoA synthase) (fatty-acid-CoA ligase), 2,3-dihydroxybenzoate-AMP ligase, probable fatty-acid-CoA ligase FADD19 (fatty-acid-CoA synthetase) (fatty-acid-CoA synthase), Serine/threonine-protein kinase pknF, Transcriptional regulatory protein devR (dosR,) Probable low molecular weight protein-tyrosine-phosphatase, Protein RecA and Mycothiol S-conjugate amidase. Particularly preferred target proteins, shown in table 1, are the proteins / gene products InhA and ThyA.

Alternatively, the target protein may be a virulence factor. Targeting and degrading such virulence factor of a bacterial cell, making such bacteria less pathogenic, may thus be another mode-of-action of bacPROTAC molecules to control bacterial infections.

As a further alternative mechanism, bacPROTAC molecules may be designed to incorporate a co-factor or other molecule that binds, physiologically, to a cellular protein, and use that co-factor or other molecule as a bacPROTAC targeting group. As a result, such bacPROTAC may result in imbalances in cell metabolism and/or other cell functions, thereby weakening the bacterial cell, making it less pathogenic, and thereby achieve control of the bacterial infection.

Another aspect relates to the compound according to the invention for use as a medicament. For the description of the compound, reference is made to the description above. Preferably, the compound is for use in the treatment of mycobacterial infections. In embodiments, the compound is for use in the treatment of infections with a mycobacteria selected from the group of *Mycobacterium tuberculosis, Mycobacterium africanum, Mycobacterium bovis, (including Bacillus Calmette-Guérin strain), Mycobacterium canetti, Mycobacterium caprae, Mycobacterium microti, Mycobacterium orygis, Mycobacterium tuberculosis* subsp. *caprae, Mycobacterium pinnipedii, Mycobacterium suricattae, Mycobacterium mungi, Mycobacterium avium subsp. avium, Mycobacterium. avium subsp. paratuberculosis, Mycobacterium avium subsp. silvaticum, Mycobacterium avium subspecies "hominissuis", Mycobacterium colombiense, Mycobacterium intracellulare, Mycobacterium yongonense;* species belonging to the clades of *Mycobacterium kansasii, Mycobacterium abscessus*/*chelonae* or *Mycobacterium fortuitum; Mycobacterium ulcerans, Mycobacterium leprae, Mycobacterium lepromatosis* and *Mycobacterium lepraemurim. Mycobacterium tuberculosis, Mycobacterium africanum, Mycobacterium bovis, (including Bacillus Calmette-Guérin strain), Mycobacterium canetti, Mycobacterium caprae, Mycobacterium microti, Mycobacterium orygis, Mycobacterium tuberculosis* subsp. *caprae, Mycobacterium pinnipedii, Mycobacterium suricattae,* and *Mycobacterium mungi* are tuberculous species and strains belonging Mycobacterium tuberculosis complex (MTBC). *M. tuberculosis* is the main cause of human tuberculosis (TB) and thus the predominant pathogen within the MTCB complex. However, M. *africanum* as well as *M. canetti,* both causing human TB, are predominantly isolated from African patients and regionally even outcompete *M. tuberculosis* in number of infections. On the other side, M. *bovis* displays a wide of spectrum of host infection, affecting human, domesticated and wild bovine, goats, elk, deer etc. Although it seems to be mostly eradicated in modern countries the number of infected domestic animals in developing countries is uncertain. Zoonotic tuberculosis is considered to be a general threat to humans, most importantly in regions where bovine tuberculosis is endemic and where people live in conditions that favour direct contact with infected animals or animal products.

*Mycobacterium avium subsp. avium, Mycobacterium. avium subsp. paratuberculosis, Mycobacterium avium subsp. silvaticum, Mycobacterium avium subspecies "hominissuis", Mycobacterium colombiense, Mycobacterium intracellulare, Mycobacterium yongonense* are tuberculous species and strains belonging to the *Mycobacterium avium* complex (MAC) except non-pathogenic *Mycobacterium indicus pranii.* Infections of non-tuberculous mycobacteria (NTM) are rising in the recent past and the number of patients with pulmonary MAC-disease is steadily increasing. Main species causing NTM in humans but also animals of livestock production is *M. avium* subsp. *avium.* However, cases caused by other species are also common. Apart of pulmonary TB, *M. avium* subsp. *paratuberculosis* is currently implicated in Crohn's disease in humans and definitively is the causative agent of Johne's disease (Paratuberculosis) in cattle and sheep. Evidence is accumulating that there is foodborne transmission from livestock production to human beings.

Other important pathogenic NTM (clades), which do not belong to MAC are:
- All species belonging to the clade of *Mycobacterium kansasii,* which often cause pulmonary infections in humans and most importantly in immunocompromised individuals.
- All species belonging to the clade of *Mycobacterium abscessus*/*chelonae,* for instance *M*. *abscessus* causes skin infection with boils or pus-filled vesicles. On the other side, *M. massiliense* belonging to this clade was for instance the source of an epidemic, post-surgery infection in Brazil in 2006 with more than 1000 infected cases.
- All species belonging to the clade of *Mycobacterium fortuitum,* for instance *M. furtuitum* is regionally the prevalent cause of NTM infection and can cause infections of many areas of the body including the skin, lymph nodes, and joints. Individuals which are immunocompromised or post-surgery are at high risk. *M. senegalense* has devastating effects on cattle in livestock production, as it is one of the two causal agents of bovine farcy.
- *Mycobacterium ulcerans* causes the "Buruli", or "Bairnsdale" ulcer (rare disease), and
- *Mycobacterium leprae, Mycobacterium lepromatosis* and *Mycobacterium lepraemurim* cause the leprosy in humans.

In embodiments, the compound is for use in the treatment of tuberculosis, preferably multidrug-resistant tuberculosis, or atypical mycobacterial infections. In embodiments, the compound is for use as an antibiotic or as an antimicrobial compound.

In embodiments, the compound can be used or included in a pharmaceutical composition. Accordingly, in another aspect the present invention relates to a pharmaceutical composition comprising as an active ingredient a compound according to the invention. The pharmaceutical composition preferably is for use in the treatment of mycobacterial infections. For the description of the compound and mycobacterial infections, reference is made to the description above. In embodiments, the pharmaceutical composition is for use in the treatment of tuberculosis, such as multidrug-resistant tuberculosis, or atypical mycobacterial infections. In embodiments, the pharmaceutical composition is for use in the treatment of infections with *Mycobacterium tuberculosis.* In embodiments, the pharmaceutical composition is for use as an antibiotic or as an antibiotic composition.

The composition may be produced under sterile conditions using standard pharmaceutical techniques well known to those skilled in the art. For compositions convenient pharmaceutical media may be employed. For example, water, glycols, oils, alcohols and the like may be used to form liquid compositions such as solutions. The composition may comprise a pharmaceutical carrier, which can be, for example, a solid, liquid, aerosol, or gas. Suitable carriers preferably are liquid and correspond to the substances ordinarily employed in formulation technology for pharmaceutical formulations.

The compounds can bind to a mycobacterial target protein and exhibit anti-mycobacterial or anti-bacterial activity either by bringing the protein into the vicinity of the mycobacterial ClpC1P1P2 protease to be degraded, but also by directly inhibiting the protein by binding. Direct inhibition would correspond to the usual mechanism of an anti-bacterial compound.

In further embodiments, the compounds can be used in screening assays of several different set-ups, for example for the screening of compounds that positively act as protein degradation inducers, or for the screening for novel mycobacterial target proteins for induced protein degradation, or for the screening for novel (chemical) targeting groups for targeting mycobacterial and particularly *M*. *tuberculosis* target proteins.

The modularity of bacPROTAC compounds, comprising a ClpCP protease-binding moiety bound to a linker which covalently links the heptapeptide to a targeting group that is capable of binding to a target protein which is to be degraded by ClpCP protease, allows to screen for (1) further compounds or moieties that bind to a ClpCP protease, (2) further targeting groups binding to suitable target proteins, and also (3) target proteins the degradation of which may be particularly effective to achieve an anti-microbial / bactericidal effect.

With respect to the screening method aspect (3), outlined in the paragraph above, the modularity of bacPROTACs allows testing the degradation of many different target proteins. Currently, over 90000 compounds are reported in the CHEMBL database that are targeting 73 different Mtb target proteins. Those 90000 compounds are potentially usable as targeting groups for the compounds of the invention: Those targeting groups may be formatted to bacPROTAC molecules, e.g. a CymA-based bacPROTAC, by linking it to a ClpC binding group via a linker, to generate a chemical bacPROTAC library. This chemical library can then be used in a screen for compounds that show a sufficiently strong antibacterial effect: e.g., a strong reduction of viability of bacterial cells used in such screen indicates that the respective compound is effective in degrading the respective target protein (which may be expected to be one of the 73 *M. tuberculosis* target proteins mentioned above), and/or that the bacterial cell is particularly sensitive to the degradation of that respective target protein. In both cases, such results will allow the skilled person to discover and develop novel bacPROTAC compounds targeting, with its targeting group, the thus identified target protein. As outlined before, the targeting groups (binders) do not have to *inhibit* the target protein in order to provide an anti-microbial effect, as known antibiotics do. Instead, it is sufficient that they *bind* to the target protein. This is a general advantage of PROTACs over small molecule inhibitors. Furthermore, as bacPROTACs do not need to modulate the target protein, but only bind to it, target proteins that so far were considered "undruggable" may now be identified as suitable targets for bacPROTAC anti-microbial compounds. This has the potential to massively increasing the pool of potential suitable targets, thereby helping to overcome the problem of acquired antibiotic resistance, which is due to the hitherto limited diversity of suitable targets for "classical" antibiotics.

Referring now to the screening method aspect (2) set out above, the bi-partite architecture of bacPROTAC compounds of the invention also allows to systematically, e.g. in the format of a chemical library, incorporate various ligands of a given Mtb target protein (the target protein being selected e.g. by the method of above aspect (3))) as a targeting group into such bacPROTAC molecules, and to determine their efficiency in inducing degradation of their cognate Mtb target protein. Again, importantly, the incorporated ligands do not need to occupy a functional site. Ligand hits that bind to the desired targets but fail to block protein function could therefore be reconsidered as bacPROTAC targeting groups, thereby i.a. significantly expanding the pool of druggable bacterial targets. In principle, the developed approach allows targeting of any essential bacterial protein and/or virulance factor proteins.

Turning to the above screening method aspect (1), the discoveries made by the inventors, and especially identification of the N-terminal domain of a ClpC protein, more specifically a mycobacterial ClpC1_{NTD}, as a target for bacPROTAC compounds, allows for the identification of additional ClpC binding ligands that may be suitable components (i.e., ClpC binding compounds) that can be used to build efficient bacPROTAC compounds.

Thus, in more detail and in accordance with the above screening method aspect (1), the invention provides a method of screening for compounds that bind to ClpCP protease, wherein the method comprises the steps of:
- providing a mycobacterial ClpCP protease;
- providing a bacPROTAC compound comprising:
   (i) a targeting group for a target protein;
   (ii) a linker; and
   (iii) a moiety that is a candidate for binding to said ClpCP protease;
- providing a cellular system or a cell lysate system that allows for ClpCP protease directed protein degradation and that comprises said target protein;
- contacting said bacPROTAC compound to be screened with said cellular system or said cell lysate system, to allow complex formation of said bacPROTAC compound with said target protein and said ClpCP protease;
- incubating said cellular system or cell lysate for an appropriate period of time; and
- determining, after said incubation period, the protein level of said target protein, whereby a reduction of said protein level, compared to a cellular system or cell lysate system to which no bacPROTAC compound has been added, is indicative for ClpCP protease directed protein degradation induced by said bacPROTAC compound, and whereby said moiety (iii) is identified as a compound that binds to said ClpCP protease.

Such compound - or moiety (iii) - that binds to said ClpCP protease, and preferably to a CLPC11_{NTD} receptor domain thereof, may then be formatted into various different bacPROTACs. I.e., linker and target group may be added or adapted, e.g. in order to target various other suitable target proteins, as explained before.

In the above screening method, the ClpCP protease is preferably a mycobacterial ClpC1P1P2 protease, and the target protein is preferably an *M. tuberculosis* target protein.

Reduction of the protein level can be monitored with usual methods such as Western blotting or capillary western immunoassay (WES^{™}-assay). The protein level can be compared to the protein level of a sample without addition of a bacPROTAC, of a sample with vehicle treatment, or to the initial protein level of the sample. Optionally, the method may include a step of isolating compounds (bacPROTAC) bound to the target protein.

The screening for compounds that act as protein degradation inducers (bacPROTACs) in embodiments may be provided as an *in vitro* degradation assay or may be provided as an *in vivo* method, e.g. using cells or cell lysates. The target protein may be provided *in vitro* in isolated form, in the form of cell lysates, or in a cellular system or environment *in vivo,* such as in mycobacterial cultures. In such embodiments, preferably wildtype, mycobacterial cultures would be incubated with the compounds. The complex of compound and target protein can be incubated with the mycobacterial ClpC1P1P2 protease in the presence of adenosine triphosphate (ATP).

The target protein may be a mycobacterial target protein. The mycobacterial target protein may be a protein from mycobacteria selected from the group of *Mycobacterium tuberculosis, Mycobacterium africanum, Mycobacterium bovis, (including Bacillus Calmette-Guérin strain), Mycobacterium canetti, Mycobacterium caprae, Mycobacterium microti, Mycobacterium orygis, Mycobacterium tuberculosis* subsp. *caprae, Mycobacterium pinnipedii, Mycobacterium suricattae, Mycobacterium mungi, Mycobacterium avium subsp. avium, Mycobacterium. avium subsp. paratuberculosis, Mycobacterium avium subsp. silvaticum, Mycobacterium avium subspecies "hominissuis", Mycobacterium colombiense, Mycobacterium intracellulare, Mycobacterium yongonense;* species belonging to the clades of *Mycobacterium kansasii, Mycobacterium abscessus*/*chelonae* or *Mycobacterium fortuitum; Mycobacterium ulcerans, Mycobacterium leprae, Mycobacterium lepromatosis* and *Mycobacterium lepraemurim.* In preferred embodiments, the target protein is a *M. tuberculosis* target protein. Preferred *Mycobacterium tuberculosis* proteins are listed in table 1. Particularly preferred are target proteins InhA and ThyA, listed in table 1.

Turning now back again to the above-mentioned screening method aspect (3), i.e. the screening for further suitable target proteins, the degradation of which may be particularly effective to achieve an anti-microbial effect, the invention provides a method of screening for target proteins for induced protein degradation, the method comprising the steps of:
- providing a bacPROTAC compound according to the invention comprising a ClpC1_{NTD}-binding synthetic cyclic heptapeptide and a targeting group for a carrier protein or protein domain;
- providing modified mycobacterial cells that express a *M. tuberculosis* target protein, such as e.g. InhA or ThyA, which is coupled to the carrier protein or protein domain;
- incubating the modified mycobacterial cells with the bacPROTAC compound; and
- analyzing the vitality of the modified mycobacterial cells, whereby a reduction of living cells is indicative for an induced degradation of the target protein, and whereby such target protein is identified as a suitable target for bacPROTAC compound induced protein degradation.

This screening method allows for informed decision making about which targets should be chosen as bacPROTAC targets and whether degradation may have advantages compared to inhibition of target proteins. The screening method allows to find targets useful as bacPROTAC targets. The vitality of mycobacterial cells, i.e. mycobacterial cell death, may be evaluated using known methods such as an MTT assay which is conventionally used to monitor a reduction of vital cells in experimental cell culture.

This screening method allows for identifying new target proteins, either by using a candidate approach using established ligands, such as from a data base, or in form of a random screen by using a random approach, making a library of bacPROTACs containing a series of small-molecule targeting groups.

In an embodiment of the screening method, the targeting group is JQ1 and the carrier protein or protein domain is BRDT. In this embodiment, a compound (sCym1 bacPROTAC) containing JQ1 as targeting group can be used to knock-down essential or other candidate proteins that are fused to BRDT to validate them as antibiotic targets. Such a screening approach may also be an interesting tool for basic research, allowing the conditional knock-out of a specific protein of interest.

The bacPROTAC-3 / BRDT protein system is usable with bacterial cells that are modified so that the cells express the BRDT protein together with one or more of the 73 *M. tuberculosis* or any other selected target protein. Upon incubating cell cultures of genetically modified *M. tuberculosis* cells with the compound (sCymA-PROTAC) comprising JQ1 as a targeting group, the mycobacterial cells will perish in case the natural mycobacterial ClpC1P1P2 protease degrades a target protein which is an essential bacterial protein. As a further advantage, this screening approach provides knowledge on further target proteins for conventional antibiotic small molecules.

More specifically, induced degradation of an endogenous protein, resulting in a relevant physiological phenotype, can be shown by using the above BRDT system to selectively degrade a fusion protein in which BRDT is linked to an essential mycobacterial protein, namely InhA or ThyA. InhA protein is a component of the fatty acid elongation system FAS-II, the target of the antituberculosis drug isoniazid. In an even more specific embodiment, InhA or ThyA will first be knocked-down in e.g. the organism M. smegmatis (essentially following the approach described in: Programmable transcriptional repression in mycobacteria using an orthogonal CRISPR interference platform. Rock JM, Hopkins FF, Chavez A, Diallo M, Chase MR, Gerrick ER, Pritchard JR, Church GM, Rubin EJ, Sassetti CM, Schnappinger D, Fortune SM. Nature Microbiology 2017). Upon depletion of InhA, cell growth will be abrogated. Thereafter, cell growth can be restored upon episomal expression of an InhA-BRDT fustion protein. Normal cell growth will indicate that such modification of InhA to an InhA-BRDT fusion protein does not interfere with its functionality. Now, the above bacPROTAC, having JQ1 as a targeting group, can be used to confirm efficiency of such bacPROTAC as a degraders, and InhA as a suitable target protein: Adding the sCym1-JQ1 bacPROTAC in this experimental system will deplete InhA(-BRDT), thereby abrogating cell growth. Various amounts of the bacPROTAC can now be titrated, allowing the determination of the apparent Minimal Inhibitory Concentration (MIC) value.

In embodiments, incubating the modified mycobacterial cells with the compound can be performed by incubating a culture of the modified mycobacterial cells with the compound, and the screening method may be an *in vivo* method. In other embodiments, incubating may be performed as an *in vitro* method, using isolated proteins or cell lysates.

In the screening method, derivatives of the compound (sCym1 bacPROTAC) can be synthesized comprising other targeting groups of known and structurally characterized ligands for targeting further target proteins. Additionally, if feasible, random screens are possible by attaching various small-molecule fragments or natural compounds to the linker and explore the effect on the mycobacterial proteome.

Finally, turning back now to the above-mentioned screening method aspect (2), i.e. the screening for further targeting groups binding to suitable target proteins, the invention provides a method of screening for targeting group for a *M. tuberculosis* target protein, the method comprising the steps of:
- providing a library of bacPROTAC compounds according to the invention comprising a ClpC1_{NTD}-binding synthetic cyclic heptapeptide and linked to a potential targeting group for a *M. tuberculosis* target protein (the targeting groups representing a variety of chemical compounds, thereby making up said library);
- providing *M. tuberculosis* mycobacterial cells comprising said *M. tuberculosis* target protein;
- incubating said *M. tuberculosis* mycobacterial cells with a compound of said library of bacPROTAC compounds; and
- determining induced degradation of the *M. tuberculosis* target protein by determining vitality of said mycobacterial cells compared to *M. tuberculosis* mycobacterial cells which were not incubated with a compound of said library of bacPROTAC compounds, whereby a reduction of said vitality of said cells upon incubation with said bacPROTAC compound is indicative of a bacPROTAC compound that includes a targeting group for a *M. tuberculosis* target protein.

The present invention also relates to the use of a compound according to the invention for the manufacture of a medicament, preferably for the manufacture of a medicament for the treatment of mycobacterial infections, particularly of tuberculosis, preferably multidrug-resistant tuberculosis, or atypical mycobacterial infections. The mycobacterial infection may be an infection with a mycobacteria is selected from the group of *Mycobacterium tuberculosis, Mycobacterium africanum, Mycobacterium bovis, (including Bacillus Calmette-Guérin strain), Mycobacterium canetti, Mycobacterium caprae, Mycobacterium microti, Mycobacterium orygis, Mycobacterium tuberculosis* subsp. *caprae, Mycobacterium pinnipedii, Mycobacterium suricattae, Mycobacterium mungi, Mycobacterium avium subsp. avium, Mycobacterium. avium subsp. paratuberculosis, Mycobacterium avium subsp. silvaticum, Mycobacterium avium subspecies "hominissuis", Mycobacterium colombiense, Mycobacterium intracellulare, Mycobacterium yongonense;* species belonging to the clades of *Mycobacterium kansasii, Mycobacterium abscessus*/*chelonae* or *Mycobacterium fortuitum; Mycobacterium ulcerans, Mycobacterium leprae, Mycobacterium lepromatosis* and *Mycobacterium lepraemurim.* For the further description of the compound and mycobacterial infections, reference is made to the description above.

A further aspect relates to a method of treating mycobacterial infections, particularly of tuberculosis, the method comprising the step of administering to a subject a therapeutically effective amount of a compound according to the invention. For the description of the compound and mycobacterial infections, reference is made to the description above.

Subjects include both human subjects and animal subjects, particularly mammalian subjects such as human subjects or mice or rats for medical purposes. The term "therapeutically effective amount" is used herein to mean an amount or dose sufficient to cause an improvement in a clinically significant condition in the subject. The method may include oral or parenteral administration.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1: *In vitro* degradation assay using the compound BacPROTAC_2
- Figure 2: *In vitro* degradation assay using *M. smegmatis* ClpC1P1P2 and the compound BacPROTAC_3.
- Figure 3: levels of BRDT_{BD1} expressed in *M. smegmatis* cells after treatment with the compound BacPROTAC_3. The bar chart shows quantification from three independent experiments normalized to BRDT_{BD1} levels before treatment (dark grey bar) and plotted as mean ± SD.
- Figure 4: Volcano plot showing the fold-change (log2) in abundance of 2912 proteins comparing the compound BacPROTAC_3 to vehicle treatment (DMSO), plotted against p-value (-log10) (triplicate analysis).

### Synthesis of non-commercial Fmoc-amino acids for the cyclomarin/cyclomarin-based bacPROTAC solid phase synthesis

### 1 Synthesis of Fmoc-L-Phe(3R-MeO)-OH

### 1.1 Phth-L-Phe(3R-OH)-OH

L-*threo*-Phenylserine (1 g, 5.52 mmol) was suspended in toluene (10 mL). Et₃N (76 µL, 0.55 mmol) and phthalic acid anhydride (815 mg, 5.52 mmol) were added and the resulting suspension was refluxed for 16 hours. The solvent was evaporated under reduced pressure and the residue was purified by silica gel chromatography using DCM/MeOH/formic acid (9:1:0.01) as the eluent system. Product containing fractions were pooled and evaporated to dryness, yielding 1.6 g (5.14 mmol, 93 %) of the desired product.

### 1.2 Phth-L-Phe(3R-MeO)-OMe

Phth-L-Phe(3*R*-OH)-OH (1.6 g, 5.14 mmol) was dissolved in dry DCM (50 mL) under an argon atmosphere. Proton sponge (11 g, 51.4 mmol) and Me₃OBF₄ (7.6 g, 51.4 mmol) were added and the resulting solution was stirred for 4 days at room temperature. The solvent was evaporated under reduced pressure to dryness and the residue was purified with silica gel column chromatography using 50-100 % EtOAc in cyclohexane as the eluent system. Product containing fractions were pooled and evaporated to dryness, yielding 1.02 g (3.01 mmol, 59 %) of the desired product.

### 1.3 Fmoc-L-Phe(3R-MeO)-OMe

Phth-L-Phe(3*R*-MeO)-OMe (1.02 g, 3.01 mmol) was dissolved in MeOH (10 mL) and the resulting solution was cooled down to 0 °C. Hydrazine monohydrate (435 µL, 13.5 mmol) was added and the resulting mixture was stirred for 4 hours at 0 °C. The solvent was evaporated under reduced pressure, yielding an oily residue. The residue was re-dissolved in a mixture of THF/water (3:1, 10 mL). Fmoc-OSu (2.53 g, 7.5 mmol) and in a minimal amount water pre-dissolved NaHCO₃ (1.26 g, 15 mmol) were added and the resulting mixture was stirred for 16 hours at room temperature. After removal of the solvent in vacuum, the residue was purified by silica gel column chromatography using 10-16 % EtOAc in cyclohexane as the eluent system. Product containing fractions were pooled and evaporated to dryness, thereby yielding 456 mg (1.06 mmol, 35 %) of the desired product.

### 1.4 Fmoc-L-Phe(3R-MeO)-OH

Fmoc-L-Phe(3*R*-MeO)-OMe (456 mg, 1.06 mmol) and Me₃SnOH (955 mg, 5.28 mmol) were dissolved in trichloroethane (10 mL). The resulting mixture was heated up for 5 h to 80 °C. The reaction was quenched by addition of aq. 5 % KHSO₄. The organic phase was separated, dried over MgSO₄ and filtrated. The solvent was removed under reduced pressure to dryness and the residue was subjected to silica gel column chromatography, using 16-50 % EtOAc in cyclohexane (acidified with 0.1% formic acid) as the eluent system. Product containing fractions were pooled and evaporated to dryness, yielding 263 mg (0.63 mmol, 60 %) of the desired product.

### 2 Synthesis of Fmoc-dehydro-isonorleucine-OH

### 2.1 (S,E)-N-Boc-glycine-(3-penten-2-yl)-ester

3-Penten-2-ol (998 mg, 11.56 mmol) and Novozym 435 (150 mg, immobilized on acryl resin, 5000 U/g) were suspended in vinylacetate (10 mL) and shaken at room temperature for 16 hours. The immobilized enzyme was filtered off and the filtrate was diluted by addition of DCM (15 mL). Boc-Gly-OH (1 g, 5.71 mmol), DIC (885 µL, 5.71 mml) and DMAP (70 mg, 0.57 mmol) were added and the resulting solution was stirred over night at room temperature. The resin was filtered off and the solvent was removed under reduced pressure. The crude product was purified by silica gel column chromatography using 16 % EtOAc in cyclohexane as the eluent system. Product containing fractions were pooled and evaporated to dryness, thereby yielding 916 mg (3.76 mmol, 33 %) of the desired product as a colorless oil.

2.2 (2*S*,3*R*,*E*)-*N*-Fmoc-2-amino-3-methylhex-4-ene acid (Fmoc-dehydro-isonorleucine-OH (*S,E*)-*N*-Boc-glycine-(3-penten-2-yl)-ester (780 mg, 3.21 mmol) and ZnCl₂ (523 mg, 3.74 mmol) were dissolved in dry THF (15 mL). This mixture was cooled down to - 78 °C. LDA (2 M in heptane/THF, 4.8 mL) was added and the reaction mixture was slowly warmed up to room temperature. After 16 hours, the reaction was quenched by the addition of aq. 5 % KHSO₄ (25 mL). The solution was extracted with EtOAc (3x 25 mL) and the pooled organic extracts were dried over MgSO₄ and filtrated. The solvent was removed under reduced pressure and the resulting residue was dissolved in 4M HCl in dioxane (10 mL). After stirring at room temperature for 1 hour, the solvent was removed under reduced pressure and the deprotected crude amino acid was dried in high vacuum. The residue was dissolved in acetonitrile:water (1:1, 20 mL), NaHCO₃ (837 mg, 9.96 mmol) and Fmoc-OSu (1.68 g, 4.98 mmol) were added and the resulting solution was stirred at room temperature for 16 hours. DCM (50 mL) and aq. 5 % KHSO₄ (50 mL) were added. The organic phase was separated, dried over MgSO₄ and filtrated. The solvent was evaporated and the obtained residue was purified by C18-RP column chromatography using 40-70 % ACN in water containing 0.1% formic acid as the eluent system. Product containing fractions were pooled and evaporated to dryness, yielding 627 mg (1.7 mmol, 53%) of the desired product as a white solid.

### 3 Synthesis of Fmoc-Trp(prenyl)-OH

### 3.1 Fmoc-L-tryptophan-methylester

Fmoc-L-Trp(Boc)-OH (5 g, 11.7 mmol) was solved in MeOH (100 mL). The solution was cooled down to 0 °C and thionyl chloride (7.5 mL, 103 mmol) was added dropwise and the resulting mixture was stirred at room temperature for 4 hours. The solvent was removed under reduced pressure and the residue was dissolved in DCM:TFA (1:1, 20 mL). After stirring for 16 hours at room temperature, brine (50 mL) was added. The organic phase was separated, dried over MgSO₄ and filtrated. After removing the solvent under reduced pressure, the crude product was obtained as a white solid (3.57 g, 8.09 mmol, 69 %) which was used in the next step without further purification.

### 3.2 Fmoc-L-N'-tert-prenyl-tryptophan-methylester

Fmoc-L-Trp-OMe (2 g, 4.54 mmol), Cu(II)OAc (1.64 g, 9.03 mmol) and silver trifluoroacetate (2 g, 9.05 mmol) were dissolved in dry acetonitrile (35 mL). Palladium(II)acetate (112 mg, 0.57 mmol) and 2-methyl-2-butene (14.5 mL, 170 mmol) were added. The resulting suspension was warmed up to 35 °C and stirred for 16 hours. More palladium(II)acetate (112 mg) was added after 60, 120 and 180 minutes. The solvent was removed under reduced pressure and purified by silica gel column chromatography using 10-16 % EtOAc in cyclohexane as the eluent system to get the desired product as a white solid (1.32 g, 2.60 mmol, 57 %).

### 3.3 Fmoc-L-N'-tert-prenyl-tryptophan (Fmoc-Trp(prenyl)-OH)

Fmoc-L-*N*'-tert-prenyl-tryptophan-methylester (516 mg, 1.02 mmol) was dissolved in TCE (10 mL). Trimethyl tin hydroxide (920 mg, 5.09 mmol) was added and the solution was stirred at 80 °C for 5 hours. The solvent was evaporated under reduced pressure and the crude product was purified by silica gel column chromatography using 25 % EtOAc in cyclohexane (acidified with 0.1% formic acid) as the eluent system to yield the desired product as a white solid (469 mg, 0.95 mmol, 93 %).

### 4 Synthesis of Fmoc-N-Me-5-Hnv(TBDMS)-OH

### 4.1 Fmoc-N-Me-Glu(OtBu)-OAll

Fmoc-N-Me-Glu(O*t*Bu)-OH (3 g, 6.8 mmol) was dissolved in DMF (10 mL). NaHCO₃ (1.43 g, 17 mmol) and allyl bromide (2.87 mL, 34 mmol) were added and the resulting solution was stirred at 50 °C for 20 hours. The solvent was removed under reduced pressure and the crude product was purified by silica gel column chromatography using 5-25 % EtOAc in cyclohexane as the eluent system to get the desired product as a colorless oil (2.6 g, 5.42 mmol, 80 %).

### 4.2 Fmoc-N-Me-Glu-OAll

Fmoc-N-Me-Glu(O*t*Bu)-OAll (2.6 g, 5.42 mmol) was dissolved in DCM:TFA 3:1 (20 mL) and stirred at room temperature for 4 hours. The solvent was removed under reduced pressure and the resulting residue was purified by C18-RP column chromatography using 40-70 % acetonitrile in water containing 0.1% formic acid as the eluent system. The desired product was obtained as a colorless oil (2.14 g, 5.05 mmol, 93 %).

### 4.3 Fmoc-N-Me-5-hydroxy-L-norvaline-OAll

Fmoc-N-Me-Glu-OAll (2.14 g, 5.05 mmol) was dissolved in THF (30 mL). Triethylamine (3.15 mL, 22.7 mmol) and isobutyl chloroformiate (2.64 mL, 20.2 mmol) were added. After 15 minutes at room temperature, NaBH₄ (1.53 g, 40.4 mmol, predissolved in 5 mL water) was added and the resulting solution was stirred for further 30 minutes at room temperature. The reaction was quenched by the addition of aq. 1 M KHSO₄ (30 mL) and was extracted with DCM (3x 25 mL). The organic phase was dried over MgSO₄, filtrated and the solvent was removed by evaporation under reduced pressure. The residue was purified by silica gel column chromatography using 5-50 % EtOAc in cyclohexane as the eluent system to get the desired product as a colorless oil (1.55 g, 3.79 mmol, 75 %).

### 4.4 Fmoc-N-Me-L-norvaline(5-OTBDMS)-OAll

Fmoc-N-Me-5-hydroxy-L-norvaline-OAll (175 mg, 0.43 mmol) was dissolved in DCM (10 mL) and DIPEA (291 µL, 1.72 mmol). TBDMS triflate (197 µL, 0.86 mmol) and DMAP (5.22 mg, 0.04 mmol) were added and the resulting mixture was stirred at room temperature for 2 hours. Aq. 5 % NaHCO₃ (10 mL) was added and the organic phase was separated, dried over MgSO₄ and filtrated. After removing the solvent under reduced pressure, the residue was purified by silica gel column chromatography using 5-25 % EtOAc in cyclohexane as the eluent system to yield the desired product as a colorless oil (197 mg, 0.37 mmol, 86 %).

### 4.5 Fmoc-N-Me-L-norvaline(5-OTBDMS)-OH (Fmoc-N-Me-5-Hnv(TBDMS)-OH)

Fmoc-N-Me-L-norvaline(5-OTBDMS)-OAll (856 mg, 1.63 mmol) was dissolved in dry DCM (10 mL). Phenylsilane (968 µL, 8.15 mmol) and tetrakis (triphenylphosphine) palladium(0) (92 mg, 0.082 mmol) were added and the reaction mixture was stirred at room temperature for 90 minutes. Brine (10 mL) was added and the separated organic phase was dried over MgSO₄ and filtrated. The solvent was removed under reduced pressure and the resulting residue was purified by C18-RP column chromatography using 40-75 % acetonitrile in water as the eluent system. The desired product was obtained as a colorless oil (554 mg, 1.15 mmol, 71 %).

### 5 Synthesis of Fmoc-Glu(TOTA-Biotin)-OH

### 5.1 Fmoc-L-Glu(TOTA-Biotin)-OtBu

Fmoc-L-Glu-O*t*Bu (425 mg, 1 mmol) and Biotin-TOTA-NH₂ (670 mg, 1.5 mmol) were dissolved in DCM (10 mL). HOBt monohydrate (306 mg, 2 mmol), DIC (309 µL, 2 mmol) and triethylamine (277 µL, 2 mmmol) were added. The resulting solution was stirred at room temperature for 16 hours. Subsequently, the organic phase was washed with aq. 5 % NaHCO₃, dried over MgSO₄ and reduced to dryness under reduced pressure. The resulting residue was purified by silica gel column chromatography, using 5 % to 25 % methanol in DCM as the eluent system. Product containing fractions were pooled and evaporated to dryness, thereby yielding 646 mg (0.76 mmol, 76 %) of the desired product as a white solid.

### 5.2 Fmoc-L-Glu(TOTA-Biotin)-OH (Fmoc-Glu(TOTA-Biotin)-OH)

Fmoc-L-Glu(TOTA-Biotin)-O*t*Bu (300 mg, 0.35 mmol) was dissolved in 4 M HCl in dioxane (4 mL) and the resulting mixture was stirred for one hour at room temperature. The solvent was removed under reduced pressure and the resulting residue was dried at high vacuum to obtain 291 mg (0.35 mmol, >98%) of the desired product as a white powder.

### 6 Synthesis of Fmoc-Glu(TOTA-(+)JQ1)-OH

### 6.1 Fmoc-L-Glu(TOTA-(+)JQ1)-OtBu

(+)-JQ1 (457 mg, 1 mmol) was dissolved in 4 M HCl in 1,4-dioxane (10 mL). The resulting solution was stirred for 4 hours at room temperature. The solvent was evaporated under reduced pressure and the resulting residue was used in the next step without further purification.

The obtained residue was dissolved in DCM (10 mL). Et₃N (693 µL, 5 mmol), HOBt monohydrate (306 mg, 2 mmol), DIC (310 µL, 2 mmol) and Boc-TOTA-NH₂ (481 mg, 1.5 mmol) were added. The resulting mixture was stirred at room temperature for 16 hours. The reaction mixture was diluted by addition saturated aq. NaHCO₃ (15 mL). The resulting phases were separated and the organic phase was washed with brine, followed by drying over MgSO₄. The solvent was removed under reduced pressure, yielding a residue that was used in the next step without further purification.

This residue was suspended in 4 M HCl in 1,4-dioxane (10 mL) and the resulting suspension was stirred for 1 hour. The solvent was removed by evaporation to dryness and the resulting residue was dissolved in DCM (10 mL), followed by addition of Et₃N (693 µL, 5 mmol), HOBt monohydrate (306 mg, 2 mmol), DIC (310 µL, 2 mmol) and Fmoc-L-Glu-O*t*Bu (638 mg, 1.5 mmol). The resulting mixture was stirred at room temperature for 16 hours. The organic phase was washed with brine, dried over MgSO₄ and evaporated under reduced pressure to dryness. The resulting residue was purified by silica gel column chromatography using 5-10% MeOH/DCM as the eluent system. Product containing fractions were pooled and evaporated to dryness, yielding 414 mg (0.41 mmol, 41%) of the desired product.

### 6.2 Fmoc-L-Glu(TOTA-(+)JQ1)-OH (Fmoc-Glu(TOTA-(+)JQ1)-OH)

Fmoc-L-Glu(TOTA-(+)JQ1)-O*t*Bu (400 mg, 0.39 mmol) was dissolved in 4 M HCl in 1,4-dioxane (10 mL) and the resulting mixture was stirred for 2 hours at room temperature. The solution was evaporated under reduced pressure to dryness to yield 389 mg (0.41 mmol, >98 %) of Fmoc-L-Glu(TOTA-(+)JQ1)-OH.

### 7 Synthesis of Fmoc-Glu(TOTA-(-)JQl)-OH

### Fmoc-L-Glu(TOTA-(-)JQ1)-OH (Fmoc-Glu(TOTA-(-)JQ1)-OH)

Fmoc-L-Glu(TOTA-(-)JQ1)-OH was synthesized in the same manner as described for the (+) enantiomer. (-)JQ1 (250 mg, 0.55 mmol) was used as starting material, delivering 273 mg of Fmoc-L-Glu(TOTA-(-)JQ1)-O*t*Bu (0.27 mmol, 49 %). From this intermediate, 260 mg (0.27 mmol, >98 %) of the desired product Fmoc-L-Glu(TOTA-(-)JQ1)-OH were obtained after deprotection.

### 8 Synthesis of Fmoc-Glu(TOTA-TMP)-OH

### 8.1 Fmoc-L-Glu(TOTA-TMP)-OtBu

*N*-(3-(2-(2-(3-Aminopropoxy)ethoxy)ethoxy)propyl)-2-(4-((2,4-diaminopyrim-idin-5-yl)methyl)-2,6-dimethoxyphenoxy)acetamide (306 mg, 0.57 mmol) was dissolved in dry DCM (10 mL). Fmoc-L-Glu-O*t*Bu (448 mg, 1.1 mmol), HOBt monohydrate (336 mg, 2.2 mmol), triethylamine (610 µL, 4.4 mmol) and DIC (340 µL, 2.2 mmol) were added. After stirring at room temperature for 16 hours, the solvent was removed under reduced pressure. The obtained residue was purified by C18-RP column chromatography using 30-75 % acetonitrile in water as the eluent system. The desired product was obtained as a white solid (360 mg, 0.38 mmol, 70 %).

### 8.2 Fmoc-L-Glu(TOTA-TMP)-OH (Fmoc-Glu(TOTA-TMP)-OH)

Fmoc-L-Glu(TOTA-TMP)-O*t*Bu (360 mg, 0.38 mmol) was dissolved in DCM:TFA (3:1, 10 mL) and stirred at room temperature for 2 hours. The solvent was removed and the residue was purified by C18-RP column chromatography using 25-40 % acetonitrile in water as the eluent system. The desired product was obtained as a white solid (243 mg, 0.27 mmol, 71 %).

### 9 Synthesis of Fmoc-Glu(TOTA-SLF')-OH

### 9.1 SLF'-TOTA-Boc

SLF' (483 mmol, 0.83 mmol) was dissolved in DCM (10 mL). Triethylamine (460 µL, 3.3 mmol), HOBt monohydrate (254 mg, 1.66 mmol), DIC (257 µL, 1.66 mmol) and Boc-TOTA (399 mg, 1.24 mmol) were added. The solution was stirred for 16 hours at room temperature. Aq. 5 % NaHCO₃ (10 mL) was added. The organic phase was separated, dried over MgSO₄ and filtrated. The solvent was removed under reduced pressure and the resulting residue was purified by silica gel column chromatography using 10-25 % MeOH/DCM as the eluent system. Product containing fractions were pooled and evaporated to dryness, yielding 615 mg (0.69 mmol, 84 %) of the desired product.

### 9.2 Fmoc-Glu(TOTA-SLF')-OtBu

SLF'-TOTA-Boc (615 mg, 0.69 mmol) was dissolved in DCM:TFA (3:1, 10 mL) and stirred at room temperature for 2 hours. The solvent was removed under reduced pressure and the obtained residue was solved in DCM (10 mL). A solution of Fmoc-Glu-O*t*Bu (364 mg, 0.85 mmol), HOBt monohydrate (175 mg, 1.14 mmol) triethylamine (237 µL, 1.7 mmol) and DIC (177 µL, 1.14 mmol) in DCM (5 mL) was added. After stirring at room temperature for 16 hours, aq. 5 % NaHCO₃ was added (10 mL). The organic phase was separated, dried over MgSO₄ and filtrated. The obtained residue was purified by silica gel column chromatography using 10-25 % MeOH/DCM as the eluent system. Product containing fractions were pooled and evaporated to dryness, yielding 690 mg (0.58 mmol, 83 %) of the desired product.

### 9.3 Fmoc-Glu(TOTA-SLF')-OH

Fmoc-Glu(TOTA-SLF')-O*t*Bu (690 mg (0.58 mmol) was dissolved in DCM:TFA (3:1, 10 mL) and stirred at room temperature for 2 hours. Brine (10 mL) was added. The organic phase was separated, dried over MgSO₄ and filtrated. The obtained residue was purified by purified by C18-RP column chromatography using 50-90 % ACN in water (acidified with 0.1% formic acid) as the eluent system. The desired product was obtained as a white solid (389 mg, 0.34 mmol, 59 %).

### 10 Synthesis of Fmoc-L-Glu(TOTA-PMX-methylester)-OH

### 10.1 Pemetrexed methylester

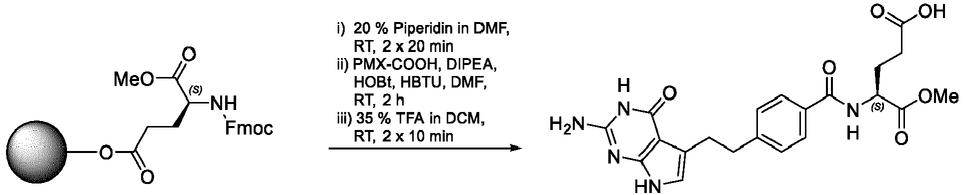

Fmoc-L-Glu-OMe (383 mg, 1 mmol) and DIPEA (510 µL, 3 mmol) was solved in DMF (10 mL). 2-chlorotrityl resin (1.25 g, max. loading 1.6 mmol g⁻¹) was added and the resulting suspension was shaken for 16 hours at room temperature.

The resin was filtered and washed 2x with DMF, 2x with DCM and 2x with DMF. A resin capping step was performed, using a mixture of DCM:MeOH:DIPEA (3:1:0.1, 10 mL) that was added to the resin and the resulting suspension was shaken for 30 minutes at room temperature. The solvent was removed and the remained resin was washed again 2x with DMF, 2x with DCM and 2x with DMF.

To cleave the Fmoc-protection group, a solution of DMF:piperidine (3:2, 10 mL) was added to the resin. The resulting suspension was shaken for 20 minutes at room temperature. The solution was removed and the cleavage step was repeated once more. The remained resin was washed 2x with DMF, 2x with DCM and 2x with DMF. 4-[2-(2-Amino-4,7-dihydro-4-oxo-3*H*-pyrrolo[2,3]pyrimidin-5-yl)ethyl]bencoic acid (447 mg, 1.5 mmol), DIPEA (341 µL, 2 mmol), HOBt monohydrate (306 mg, 2 mmol) and HBTU (759 mg, 2 mmol) were solved in DMF (10 mL) and stirred 15 minutes at room temperature. This solution was than added to the resin and the resulting suspension was shaken at room temperature. After 2 hours, the resin was washed 5x with DCM and a mixture of DCM:TFA (3:1, 10 mL) was added to the resin. The suspension was shaken for 10 minutes at room temperature. This step was repeated once more time. After washing the resin with DCM (3 x 10 mL), the cleaving and the washing solutions were combined and the solvent was removed under reduced pressure to obtain pemetrexed methylester as a pale rose solid (429 mg, 0.97 mmol, 97 %), that was used in the next step without further purification.

### 10.2 Boc-TOTA-pemetrexed methylester

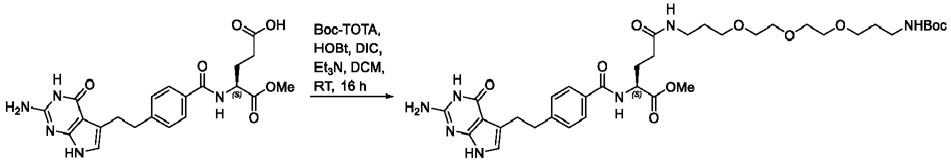

Pemetrexed methylester (420 mg, 0.95 mmol), triethylamine (263 µL, 1.9 mmol), HOBt monohydrate (214 mg, 1.4 mmol), DIC (294 µL, 1.9 mmol) and Boc-TOTA (456 mg, 1.4 mmol) were dissolved in DMF (5 mL) and stirred at room temperature for 16 hours. The solvent was removed under reduced pressure and the obtained residue was purified by C18-RP-chromatography (25 % → 50 % ACN in water). The desired product was obtained as a green solid (406 mg, 0.55 mmol, 58 %).

### 10.3 Fmoc-L-Glu(TOTA-PMX-methylester)-OH

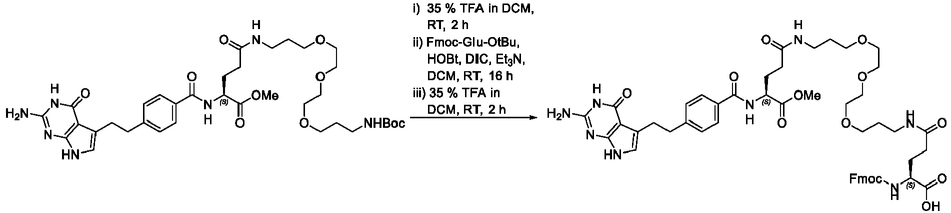

The Boc-protected amine was solved in a mixture of DCM:TFA (3:1, 5 mL) and stirred at room temperature. After 2 hours, the solvent was removed under reduced pressure.

The resulting residue was solved in DCM (10 mL). Fmoc-*L*-Glu-O*t*Bu (281 mg, 0.66 mmol), HOBt monohydrate (168 mg, 1.1 mmol), DIC (170 µL, 1.1 mmol) and triethylamine (305 µL, 2.2 mmol) were added and the resulting solution was stirred at room temperature for 16 hours. The reaction was quenched by the addition of aq. 5 % NaHCO₃ (10 mL). The organic phase was separated, dried over MgSO₄ and filtrated.

After removing the solvent under reduced pressure, the obtained residue was solved in a mixture of DCM:TFA (3:1, 5 mL) and stirred at room temperature for 2 hours. The solvent was evaporated and the remaining residue was purified by C18-RP-chromatography (30 % → 70 % ACN in water). The desired product was obtained as a blue solid (382 mg, 0.38 mmol, 69 %).

### Synthesis protocols

The synthesis of the cyclomarin derivatives including cyclomarin-based BacPROTACs was achieved by a combination of solid phase peptide synthesis and solution phase synthesis procedures using a set of general procedures.

### General procedure 1: Loading of the resin with the first amino acid

The corresponding Fmoc-amino acid derivative (exact amounts are given in the case examples) and N,N-Diisopropylethylamine (DIPEA) (2 eq.) were dissolved in dimethylformamide (DMF) (5 mL). The resulting solution was added to 2-chlorotrityl resin (1.6 mmol g⁻¹ initial loading). The resulting suspension was shaken for 16 h at room temperature. After removal of the solution, the resin was washed 2x with DMF, 2x with Methylene chloride (DCM) and 2x with DMF. A subsequent resin capping step was performed, using a mixture of DCM:MeOH:DIPEA (3:1:0.1, 4 mL) that was added to the resin and the resulting suspension was shaken for 30 minutes at room temperature. The solvent was removed and the remained resin was washed again 2x with DMF, 2x with DCM and 2x with DMF.

### General procedure 2: Assembly of the cyclomarin sequence on solid phase

The open-chain cyclomarin peptide was built up by application of three generic solid phase peptide synthesis procedures that were used iteratively:
Fmoc deprotection. A solution of DMF:piperidine (3:2, 5 mL) was added to the resin. The resulting suspension was shaken for 20 minutes at room temperature. The solution was removed and the cleavage step was repeated once more with the same reagents. The remained resin was washed 2x with DMF, 2x with DCM and 2x with DMF.
Coupling of Fmoc-protected amino acids. The corresponding Fmoc-protected amino acid (3 eq.), HOBt monohydrate (3 eq.), HBTU (3 eq.) and DIPEA (3 eq.) were dissolved in DMF (5 mL). This solution was added to the resin and the resulting suspension was shaken for 2 hours at room temperature. The solution was removed and the remained resin was washed 2x with DMF, 2x with DCM and 2x with DMF.

Alternative coupling procedure for coupling of Fmoc-amino acids on resin-bound N-methyl amino acids. The corresponding Fmoc-protected amino acid (3 eq.), 2-Bromo-1-ethyl-pyridinium tetrafluoroborate (3 eq.) and DIPEA (6 eq.) were dissolved in DMF (5 mL). This solution was added to the resin and the resulting suspension was shaken at room temperature for 4 hours. The solution was removed and the remained resin was washed 2x with DMF, 2x with DCM and 2x with DMF.

### General procedure 3: Cleavage of the open-chain cyclomarin precursor from the resin

A mixture of DCM:hexafluoroisopropanol (3:1, 5 mL) was added to the resin. The resulting suspension was shaken at room temperature for 20 min. The cleavage solution was removed from the resin and transferred into a flask. The resin was washed 3× with DCM (2 mL) and the washing solutions were combined with the cleavage solution. The resulting solution was evaporated under reduced pressure to dryness to obtain the linear peptide that was used in the next step without further purification.

### General procedure 4: In solution cyclization

For the cyclization of the open-chain precursor, two different cyclization methods were used:
Method A. The cleaved peptide was dissolved in DCM (final concentration: 0.75 mM). DIPEA (8 eq.) and 50% propanephosphonic acid anhydride (4 eq., dissolved in ethyl acetate) were added. The resulting solution was stirred at room temperature for one hour. Brine (100 mL) was added, the organic phase was separated, dried over MgSO₄ and the organic phase was removed under reduced pressure to obtain the cyclized peptide which was used in the next step without further purification.
Method B. The cleaved peptide was dissolved in DCM (final concentration: 0.75 mM). Triethylamine (3 eq.), HOAt (3 eq.) and DIC (3 eq.) were added. The resulting solution was stirred at room temperature for 16 hours. The reaction was quenched by the addition of aq. 5 % NaHCO₃ (100 mL). The organic phase was separated, dried over MgSO₄ and removed under reduced pressure to obtain the cyclized peptide that was used in the next step without further purification.

### General procedure 5: Protecting group cleavage and purification

Three different methods were used for cleaving off the remaining protecting groups and final purification:
Method A. The cyclized peptide was dissolved in a TFA:DCM (3:1, 5 mL) mixture and the resulting solution was stirred at room temperature for 16 h. The solvent was removed by evaporation and the resulting residue was purified by RP-HPLC with 0.1% TFA in water and 0.1% TFA in acetonitrile as eluent systems. Product containing fractions were pooled and lyophilized to obtain the desired peptide as a white powder.
Method B. The cyclized peptide was dissolved in dry DCM (5 mL). After the addition of 2,6-lutidine (20 eq.), the resulting solution was cooled down to 0 °C. TMSOTf (16 eq.) was added. After 15 minutes, the reaction mixture was slowly warmed up to room temperature and stirred for 16 hours. Brine (100 mL) was added, the organic phase was separated, dried over MgSO₄ and removed under reduced pressure. The resulting residue was purified by RP-HPLC with 0.1% TFA in water and 0.1% TFA in acetonitrile as eluent systems. Product containing fractions were pooled and lyophilized to obtain the desired peptide as a white powder.
Method C. The cyclized peptide was dissolved in 1 M TBAF in THF (5 mL). After stirring for 2 hours at room temperature, DCM (10 mL) was added. Aq. 5 % NaHCO₃ was added, the organic phase was separated, dried over MgSO₄ and removed under reduced pressure. The residue was dissolved in THF and 25 % KOH in MeOH (1 mL) was added. After stirring for 16 hours at room temperature, the solvent was removed under reduced pressure. The resulting residue was purified by RP-HPLC with 0.1% TFA in water and 0.1% TFA in acetonitrile as eluent systems. Product containing fractions were pooled and lyophilized to obtain the desired peptide as a white powder.
Method D. The cyclized peptide was purified by RP-HPLC with 0.1% TFA in water and 0.1% TFA in acetonitrile as eluent systems without prior protecting group cleavage. Product containing fractions were pooled and lyophilized to obtain the desired peptide as a white powder.

### Example 1: Synthesis of cyclic heptapeptides

The generic methodologies as described above were used to synthesize the following cyclomarin analogues.

### Example 1.1: CycloA_D2

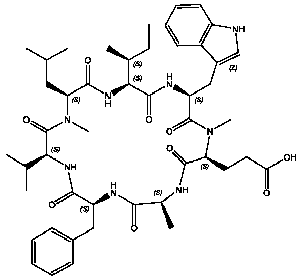

250 mg 2-chlorotrityl resin (corresponding to 0.40 mmol initial loading) were loaded with 171 mg (0.44 mmol) Fmoc-L-Phe-OH according to general procedure 1. The linear peptide was synthesized according to general procedure 2 (using the amino acids Fmoc-Ala-OH, Fmoc-N-Me-Glu(tBu)-OH, Fmoc-Trp(Boc)-OH, Fmoc-Ile-OH, Fmoc-N-Me-Leu-OH, Fmoc-Val-OH) and 3, yielding 340 mg (0.320 mmol, 80 %) of the open-chain precursor. Of this peptide, 197 mg (0.185 mmol) were cyclized according to general procedure 4 - method A, yielding 99 mg (0.095 mmol, 52 %) of the cyclized peptide. 45 mg (0.043 mmol) of this cyclized peptide were then deprotected and purified via general procedure 5 - method A, thereby yielding 11.5 mg (0.013 mmol, 29 %) of the desired product.

### Example 1.2: CycloA_D3

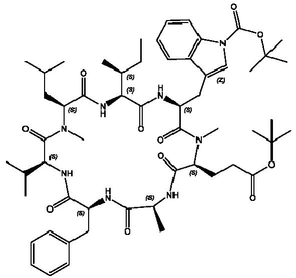

250 mg 2-chlorotrityl resin (corresponding to 0.40 mmol initial loading) were loaded with 171 mg (0.44 mmol) Fmoc-L-Phe-OH according to general procedure 1. The linear peptide was synthesized according to general procedure 2 (using the amino acids Fmoc-Ala-OH, Fmoc-N-Me-Glu(tBu)-OH, Fmoc-Trp(Boc)-OH, Fmoc-Ile-OH, Fmoc-N-Me-Leu-OH, Fmoc-Val-OH) and 3, yielding 340 mg (0.320 mmol, 80 %) of the open-chain precursor. Of this peptide, 140 mg (0.13 mmol) were cyclized according to general procedure 4 - method A, yielding 72 mg (0.07 mmol, 54 %) of the cyclized peptide. This cyclized peptide was then purified by a modified general procedure 5 - method D, thereby yielding 53 mg (0.51 mmol, 39 %) of the desired product.

### Example 1.3: CycloA_D4

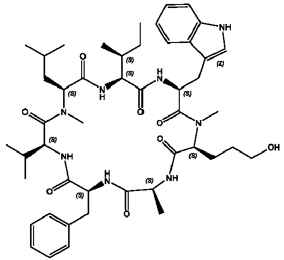

125 mg 2-chlorotrityl resin (corresponding to 0.2 mmol initial loading) were loaded with 171 mg (0.40 mmol) Fmoc-L-Phe-OH according to general procedure 1. The linear peptide was synthesized according to general procedure 2 (using the amino acids Fmoc-Ala-OH, Fmoc-N-Me-5-Hnv(TBDMS)-OH, Fmoc-Trp(Boc)-OH, Fmoc-Ile-OH, Fmoc-N-Me-Leu-OH, Fmoc-Val-OH) and 3, yielding 85 mg (0.077 mmol, 39 %) of the open-chain precursor. Of this peptide, 20 mg (0.018 mmol) were cyclized according to general procedure 4 - method A, yielding 19 mg (0.017 mmol, 94 %) of the cyclized peptide. This cyclized peptide was then deprotected and purified via general procedure 5 - method C, yielding 6 mg (0.006 mmol, 35 %) of the desired product.

### Example 1.4: CycloA_D5a

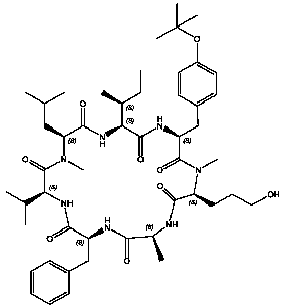

250 mg 2-chlorotrityl resin (corresponding to 0.40 mmol initial loading) were loaded with 171 mg (0.44 mmol) Fmoc-L-Phe-OH according to general procedure 1. The linear peptide was synthesized according to general procedure 2 (using the amino acids Fmoc-Ala-OH, Fmoc-N-Me-Glu(Me)-OH, Fmoc-Tyr(tBu)-OH, Fmoc-Ile-OH, Fmoc-N-Me-Leu-OH, Fmoc-Val-OH) and 3, yielding 171 mg (0.172 mmol, 43 %) of the open-chain precursor. This peptide was cyclized according to general procedure 4 - method A, yielding 121 mg (0.124 mmol, 31 %) of the cyclized peptide. Of this peptide, 80 mg (0.082 mmol) were dissolved in 1,4-dioxane:water (1:1, 5 mL). NaBH₄ (31 mg, 0.82 mmol) was added and the resulting mixture was stirred at room temperature for 7 days. DCM (10 mL) and brine (10 mL) were added and the organic phase was separated, dried over MgSO₄ and removed under reduced pressure. The resulting peptide was purified by general procedure 5 - method D, yielding 15 mg (0.017 mmol, 21 %) of the desired product.

### Example 1.5: CycloA_D5b

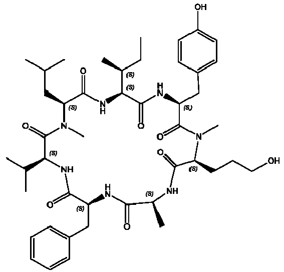

CycloA_D5a (10 mg, 0.011 mmol) was dissolved in 4 M HCl in 1,4-dioxane (5 mL) and stirred for 1 h at room temperature. The solvent was removed under reduced pressure and the crude product was purified by HPLC using general procedure 5 - method D, thereby yielding 9 mg (0.0099 mmol, 90 %) of the desired product.

### Example 1.6: CycloA_D6

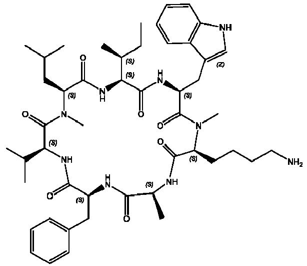

150 mg 2-chlorotrityl resin (corresponding to 0.24 mmol initial loading) were loaded with 171 mg (0.44 mmol) Fmoc-L-Phe-OH according to general procedure 1. The linear peptide was synthesized according to general procedure 2 (using the amino acids Fmoc-Ala-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 191 mg (0.173 mmol, 72 %) of the open-chain precursor. This peptide was cyclized according to general procedure 4 - method A, yielding 142 mg (0.131 mmol, 75 %) of the cyclized peptide. Of this peptide, 50 mg (0.046 mmol) peptide were deprotected and final purified via general procedure 5 - method A, yielding 17 mg (0.019 mmol, 41 %) of the desired product.

### Example 1.7: CycloA_D7

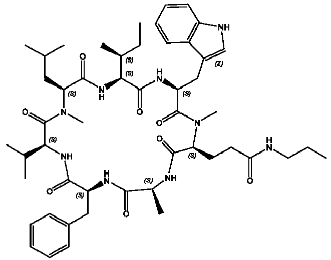

CycloA_D2 (40 mg, 0.045 mmol) was dissolved in DCM (5 mL). DIPEA (15.3 µL, 0.09 mmol), propyl amine (7.4 µL, 0.09 mmol), HOBt monohydrate (13.8 mg, 0.09 mmol) and EDCI.HCl (17.2 mg, 0.09 mmol) were added. The resulting solution was stirred at room temperature for 16 hours. Aq. 5 % NaHCO₃ (5 mL) was added and the organic phase was separated, dried over MgSO₄ and removed under reduced pressure. The remaining residue was purified according to general procedure 5 - method D to yield 19 mg (0.020 mmol, 44 %) of the desired product.

### Example 1.8: CycloA_D9

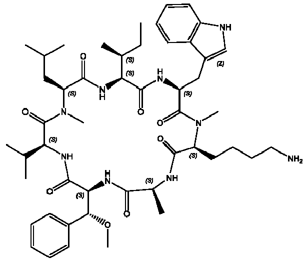

150 mg 2-chlorotrityl resin (corresponding to 0.24 mmol initial loading) were loaded with 209 mg (0.50 mmol) Fmoc-L-Phe(3*R*-MeO)-OH according to general procedure 1. The linear peptide was synthesized according to general procedure 2 (using the amino acids Fmoc-Ala-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 156 mg (0.138 mmol, 58 %) of the open-chain precursor. This peptide was cyclized according to general procedure 4 - method A, yielding 135 mg (0.121 mmol, 88 %) of the cyclized peptide. Of this peptide, 52 mg (0.047 mmol) peptide were deprotected and finally purified via general procedure 5 - method A to yield 7 mg (0.007 mmol, 16 %) of the desired product.

### Example 1.9: CycloA_D10

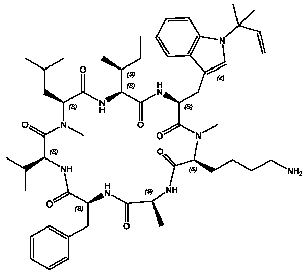

300 mg 2-chlorotrityl resin (corresponding to 0.48 mmol initial loading) were loaded with 310 mg (0.80 mmol) Fmoc-L-Phe-OH according to general procedure 1. The linear peptide was synthesized according to general procedure 2 (using the amino acids Fmoc-Ala-OH, Fmoc-N-Me-Lys(Boc)-OH, Fmoc-Trp(Prenyl)-OH, Fmoc-Ile-OH, Fmoc-N-Me-Leu-OH, Fmoc-Val-OH) and 3, yielding 216 mg (0.201 mmol, 42 %) of the open-chain precursor. This peptide was cyclized according to general procedure 4 - method B, yielding 164 mg (0.156 mmol, 78 %) of the cyclized peptide. Of this peptide, 49 mg (0.047 mmol) peptide were deprotected and purified via general procedure 5 - method B to yield 5 mg (0.005 mmol, 11 %) of the desired product.

### Example 1.10: CycloA_D11

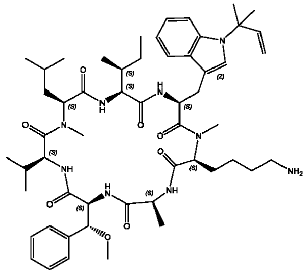

150 mg 2-chlorotrityl resin (corresponding to 0.24 mmol initial loading) were loaded with 140 mg (0.33 mmol) Fmoc-L-Phe(3*R*-MeO)-OH according to general procedure 1. The linear peptide was synthesized according to general procedure 2 (using the amino acids Fmoc-Ala-OH, Fmoc-N-Me-Lys(Boc)-OH, Fmoc-Trp(Prenyl)-OH, Fmoc-Ile-OH, Fmoc-N-Me-Leu-OH, Fmoc-Val-OH) and 3, yielding 129 mg (0.120 mmol, 50 %) of the open-chain precursor. This peptide was cyclized according to general procedure 4 - method B, yielding 93 mg (0.079 mmol, 65 %) of the cyclized peptide. This peptide was deprotected and purified via general procedure 5 - method B to yield 9.7 mg (0.010 mmol, 12 %) of the desired product.

### Example 1.11: CycloA_D13

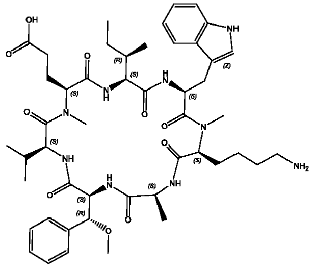

150 mg 2-chlorotrityl resin (corresponding to 0.24 mmol initial loading) were loaded with 125 mg (0.30 mmol) Fmoc-L-Phe(3*R*-MeO)-OH according to general procedure 1. The linear peptide was synthesized according to general procedure 2 (using the amino acids Fmoc-Ala-OH, Fmoc-N-Me-Lys(Boc)-OH, Fmoc-Trp(Boc)-OH, Fmoc-Ile-OH, Fmoc-N-Me-Glu(tBu)-OH, Fmoc-Val-OH) and 3, yielding 156 mg (0.129 mmol, 46 %) of the open-chain precursor. This peptide was cyclized according to general procedure 4 - method A, yielding 80 mg (0.067 mmol, 52 %) of the cyclized peptide. This peptide was deprotected and purified via general procedure 5 - method A to yield 13.7 mg (0.014 mmol, 21 %) of the desired product.

### Example 1.12: CycloA_D14

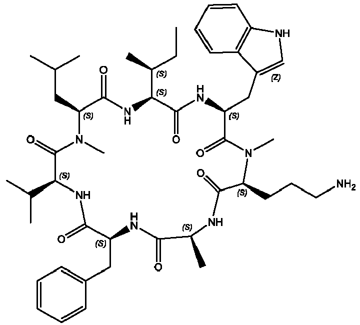

150 mg 2-chlorotrityl resin (corresponding to 0.24 mmol initial loading) were loaded with 155 mg (0.40 mmol) Fmoc-L-Phe-OH according to general procedure 1. The linear peptide was synthesized according to general procedure 2 (using the amino acids Fmoc-Ala-OH, Fmoc-N-Me-Orn(Boc)-OH, Fmoc-Trp(Boc)-OH, Fmoc-Ile-OH, Fmoc-N-Me-Leu-OH, Fmoc-Val-OH) and 3, yielding 155 mg (0.142 mmol, 59 %) of the open-chain precursor. Of this peptide, 55 mg (0.050 mmol) were cyclized according to general procedure 4 - method A, yielding 62 mg (0.058 mmol, >98 %) of the cyclized peptide. This peptide was deprotected and purified via general procedure 5 - method A to yield 12.6 mg (0.014 mmol, 28 %) of the desired product.

### Example 1.13: CycloA_D15a

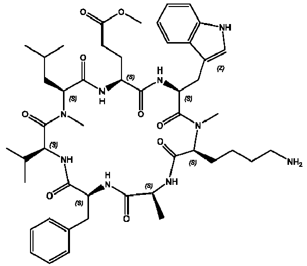

200 mg 2-chlorotrityl resin (corresponding to 0.32 mmol initial loading) were loaded with 350 mg (0.90 mmol) Fmoc-L-Phe-OH according to general procedure 1. The linear peptide was synthesized according to general procedure 2 (using the amino acids Fmoc-Ala-OH, Fmoc-N-Me-Lys(Boc)-OH, Fmoc-Trp(Boc)-OH, Fmoc-Glu(Me)-OH, Fmoc-N-Me-Leu-OH, Fmoc-Val-OH) and 3, yielding 110 mg (0.096 mmol, 30 %) of the open-chain precursor. This peptide was cyclized according to general procedure 4 - method A, yielding 105 mg (0.094 mmol, 97 %) of the cyclized peptide. Of this peptide, 30 mg (0.027 mmol) were deprotected and purified via general procedure 5 - method A to yield 11 mg (0.012 mmol, 44 %) of the desired product.

### Example 1.14: CycloA_D15b

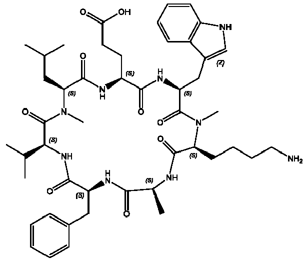

200 mg 2-chlorotrityl resin (corresponding to 0.32 mmol initial loading) were loaded with 350 mg (0.90 mmol) Fmoc-L-Phe-OH according to general procedure 1. The linear peptide was synthesized according to general procedure 2 (using the amino acids Fmoc-Ala-OH, Fmoc-N-Me-Lys(Boc)-OH, Fmoc-Trp(Boc)-OH, Fmoc-Glu(Me)-OH, Fmoc-N-Me-Leu-OH, Fmoc-Val-OH) and 3, yielding 110 mg (0.096 mmol, 30 %) of the open-chain precursor. This peptide was cyclized according to general procedure 4 - method A, yielding 105 mg (0.094 mmol, 97 %) of the cyclized peptide. Of this peptide, 60 mg (0.054 mmol) peptide were dissolved in TCE (5 mL). Trimethyl tin hydroxide (1.08 mmol) was added and the resulting solution was heated up to 80 °C for 24 hours. The crude reaction mixture was diluted with aq. 5 % KHSO₄. The organic phase was separated, dried over MgSO₄ and removed under reduced pressure. The resulting residue was deprotected and purified via general procedure 5 - method A to yield 17 mg (0.019 mmol, 35 %) of the desired product.

### Example 1.15: CycloA_D17

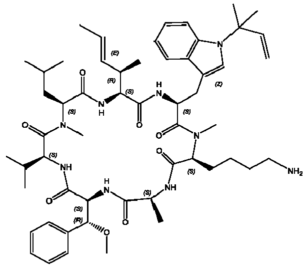

150 mg 2-chlorotrityl resin (corresponding to 0.24 mmol initial loading) were loaded with 150 mg (0.36 mmol) Fmoc-L-Phe(3*R*-MeO)-OH according to general procedure 1. The linear peptide was synthesized according to general procedure 2 (using the amino acids Fmoc-Ala-OH, Fmoc-N-Me-Lys(Boc)-OH, Fmoc-Trp(prenyl)-OH, Fmoc-dehydro-isonorleucine-OH, Fmoc-N-Me-Leu-OH, Fmoc-Val-OH) and 3, yielding 111 mg (0.10 mmol, 42 %) of the open-chain precursor. This peptide was cyclized according to general procedure 4 - method B, yielding 89 mg (0.081 mmol, 81 %) of the cyclized peptide. Of this peptide, 30 mg (0.027 mmol) were deprotected and purified via general procedure 5 - method B to yield 10 mg (0.010 mmol, 12 %) of the desired product.

### Example 2: Synthesis of cyclic heptapeptides bound to a linker and a targeting group

The Cyclomarin-based bacPROTACs were analoguesly synthesized.

### Example 2.1: Cym_bacPROTAC_1

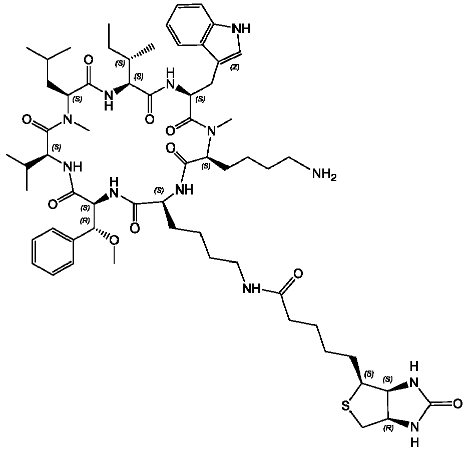

150 mg 2-chlorotrityl resin (corresponding to 0.24 mmol initial loading) were loaded with 150 mg (0.36 mmol) Fmoc-L-Phe(3*R*-MeO)-OH according to general procedure 1. The linear peptide was synthesized according to general procedure 2 (using the amino acids Fmoc-Lys(Biotin)-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 149 mg (0.113 mmol, 47 %) of the open-chain precursor. This peptide was cyclized according to general procedure 4 - method B, yielding 105 mg (0.081 mmol, 72 %) of the cyclized peptide. This peptide was then deprotected and purified via general procedure 5 - method A to yield 15 mg (0.012 mmol, 15 %) of the desired product.

### Example 2.2: Cym_bacPROTAC_2

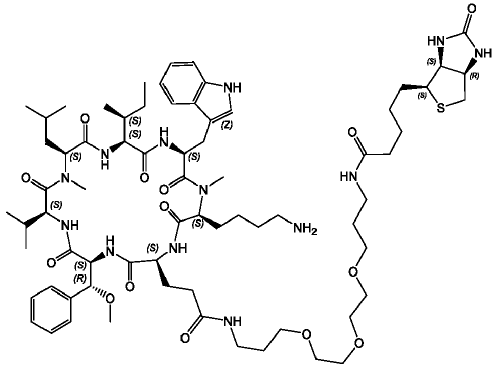

200 mg 2-chlorotrityl resin (corresponding to 0.32 mmol initial loading) were loaded with 105 mg (0.25 mmol) Fmoc-L-Phe(3*R*-MeO)-OH according to general procedure 1. The linear peptide was synthesized according to general procedure 2 (using the amino acids Fmoc-Glu(TOTA-Biotin)-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 269 mg (0.167 mmol, 67 %) of the open-chain precursor. This peptide was cyclized according to general procedure 4 - method B, yielding 169 mg (0.105 mmol, 63 %) of the cyclized peptide. This peptide was then deprotected and purified via general procedure 5 - method A to yield 12.7 mg (0.009 mmol, 9 %) of the desired product.

### Example 2.3: Cym_bacPROTAC_3

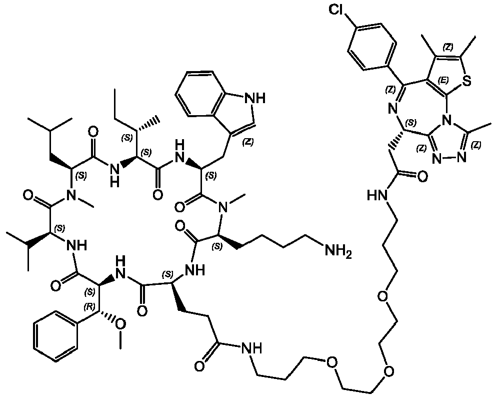

200 mg 2-chlorotrityl resin (corresponding to 0.32 mmol initial loading) were loaded with 105 mg (0.25 mmol) Fmoc-L-Phe(3*R*-MeO)-OH according to general procedure 1. The linear peptide was synthesized according to general procedure 2 (using the amino acids Fmoc-Glu(TOTA-(+)JQ1)-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 229 mg (0.137 mmol, 55 %) of the open-chain precursor. This peptide was cyclized according to general procedure 4 - method B, yielding 146 mg (0.088 mmol, 64 %) of the cyclized peptide. This peptide was then deprotected and purified via general procedure 5 - method A to yield 42.5 mg (0.027 mmol, 31 %) of the desired product.

### Example 2.4: Cym_bacPROTAC_4

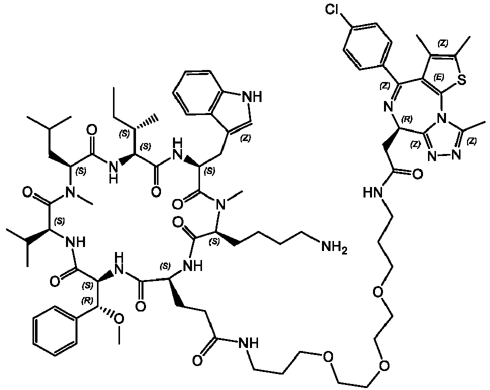

100 mg 2-chlorotrityl resin (corresponding to 0.16 mmol initial loading) were loaded with 53 mg (0.125 mmol) Fmoc-L-Phe(3*R*-MeO)-OH according to general procedure 1. The linear peptide was synthesized according to general procedure 2 (using the amino acids Fmoc-Glu(TOTA-(-)JQ1)-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 89 mg (0.050 mmol, 40 %) of the open-chain precursor. This peptide was cyclized according to general procedure 4 - method B, yielding 61 mg (0.035 mmol, 70 %) of the cyclized peptide. This peptide was then deprotected and purified via general procedure 5 - method A to yield 3.3 mg (0.002 mmol, 10 %) of the desired product.

### Example 2.5: Cym_bacPROTAC_5

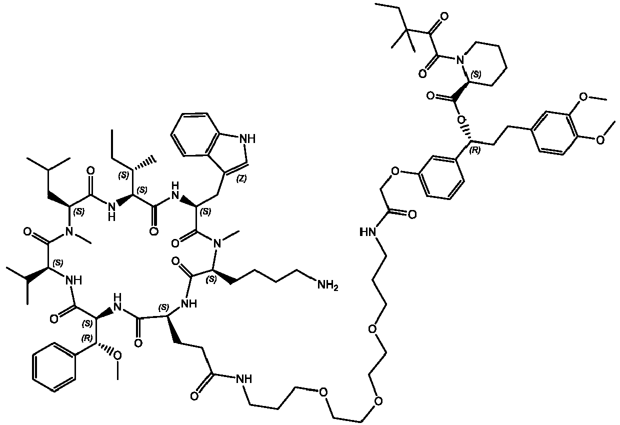

200 mg 2-chlorotrityl resin (corresponding to 0.32 mmol initial loading) were loaded with 105 mg (0.25 mmol) Fmoc-L-Phe(3*R*-MeO)-OH according to general procedure 1. The linear peptide was synthesized according to general procedure 2 (using the amino acids Fmoc-Glu(TOTA-SLF')-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 345 mg (0.176 mmol, 70 %) of the open-chain precursor. This peptide was cyclized according to general procedure 4 - method B, yielding 183 mg (0.094 mmol, 54 %) of the cyclized peptide. This peptide was then deprotected and purified via general procedure 5 - method A to yield 6.4 mg (0.004 mmol, 15 %) of the desired product.

### Example 2.6: Cym_bacPROTAC_6

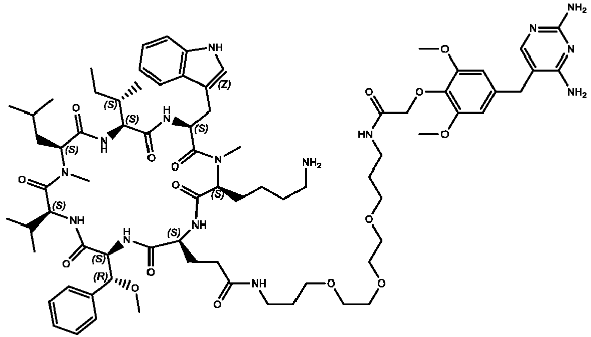

200 mg 2-chlorotrityl resin (corresponding to 0.32 mmol initial loading) were loaded with 121 mg (0.25 mmol) Fmoc-L-N-Me-Lys(Boc)-OH according to general procedure 1. The linear peptide was synthesized according to general procedure 2 (using the amino acids Fmoc-Glu(TOTA-TMP)-OH, Fmoc-N-Me-Lys(Boc)-OH, Fmoc-Trp(Boc)-OH, Fmoc-Ile-OH, Fmoc-N-Me-Leu-OH, Fmoc-Val-OH) and 3, yielding 207 mg (0.121 mmol, 48 %). Of this peptide, 100 mg (0.058 mmol) were cyclized according to general procedure 4 - method B, yielding 65 mg (0.038 mmol, 65 %) of the cyclized peptide. This peptide was then deprotected and purified via general procedure 5 - method A to yield 11 mg (0.007 mmol, 19 %) of the desired product.

### Example 2.7: Cym_bacPROTAC_7

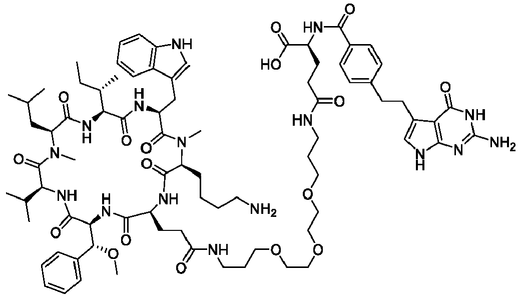

125 mg 2-chlorotrityl resin (corresponding to 0.2 mmol initial loading) were loaded with 71 mg (0.15 mmol) Fmoc-L-N-Me-Lys(Boc)-OH according to general procedure 1.
The linear peptide was synthesized according to general procedure 2 (using the amino acids Fmoc-*L-*N-Me-Lys(Boc)-OH, Fmoc-*L*-Trp(Boc)-OH, Fmoc-*L*-Ile-OH, Fmoc-N-Me-*L*-Leu-OH, Fmoc-*L*-Val-OH, Fmoc-*L*-Phe(3*R*-MeO)-OH and Fmoc-*L*-Glu(TOTA-PMX-methylester)-OH) and 3, yielding 64 mg (0.035 mmol, 23 %).
Of this peptide, 64 mg (0.035 mmol) were cyclized according to general procedure 4 - method B, yielding 49 mg (0.027 mmol, 77 %) of the cyclized peptide.
The cyclized peptide (49 mg, 0.027 mmol) was solved in DCM:TFA (3:1, 5 mL) and stirred at room temperature for 2 hours. After removing the solvents under reduced pressure, the obtained residue (37 mg) was dissolved in THF:MeOH:H₂O (3:1:1, 5 mL). Lithium monohydrate (4.8 mg, solved in 500 µL water) was added and the resulting reaction mixture was stirred at room temperature for 72 hours.
The solvent was removed under reduced pressure and the remaining residue was purified via general procedure 5 - method A to yield 4.4 mg (0.003 mmol, 12 %) of the desired product.

### Example 2.8: Cym_bacPROTAC_8

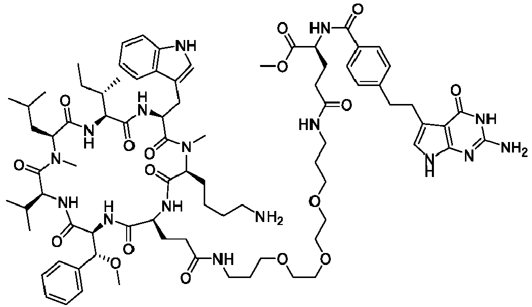

200 mg 2-chlorotrityl resin (corresponding to 0.32 mmol initial loading) were loaded with 118 mg (0.25 mmol) Fmoc-L-N-Me-Lys(Boc)-OH according to general procedure 1.
The linear peptide was synthesized according to general procedure 2 (using the amino acids Fmoc-L-N-Me-Lys(Boc)-OH, Fmoc-L-Trp(Boc)-OH, Fmoc-L-Ile-OH, Fmoc-N-Me-L-Leu-OH, Fmoc-L-Val-OH, Fmoc-*L*-Phe(3*R*-MeO)-OH and Fmoc-L-Glu(TOTA-PMX-methylester)-OH) and 3, yielding 160 mg (0.088 mmol, 35 %).
Of this peptide, 64 mg (0.035 mmol) were cyclized according to general procedure 4 - method B, yielding 93 mg (0.051 mmol, 58 %) of the cyclized peptide.
This peptide was then deprotected and purified via general procedure 5 - method A to yield 3.6 mg (0.002 mmol, 9 %) of the desired product.

### Example 2.9: Cym_bacPROTAC_9

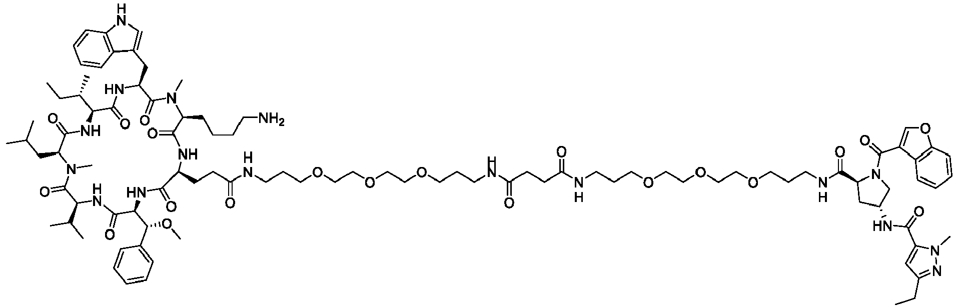

Cym_bacPROTAC_9 was synthesized *via* a solid phase peptide synthesis approach. Prior to solid phase peptide synthesis, a further non-commercial amino acid building block was synthesized. 2.9.1 Synthesis of (2*S*,4*R*)-1-(benzofuran-3-carbonyl)-4-(3-ethyl-1-methyl-1*H*-pyrazole-5-carboxamido)pyrrolidine-2-carboxylic acid

(2*S*,4*R*)-1-(benzofuran-3-carbonyl)-4-(3-ethyl-1-methyl-1*H*-pyrazole-5-carboxamido)pyrrolidine-2-carboxylic acid was synthesized according to literature (J. Med. Chem. 2014, 57, 1276-1288).

### 2.9.2 InhA-conjugate 1

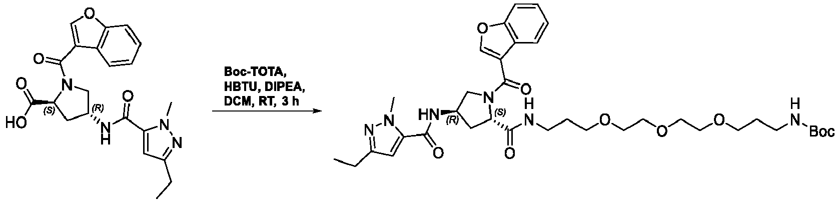

(2*S*,4*R*)-1-(benzofuran-3-carbonyl)-4-(3-ethyl-1-methyl-1*H*-pyrazole-5-carboxamido)pyrrolidine-2-carboxylic acid was (205.2 mmol, 0.5 mmol) was solved in DCM (5 mL). HBTU (227.5 mg, 0.6 mmol) and DIPEA (255.1 µL, 1.5 mmol) were added and the resulting solution was stirred at room temperature. After 15 minutes, Boc-TOTA (192.3 mg, 0.6 mmol) was added and the reaction mixture was stirred at room temperature for three hours.
The organic phase was subsequently washed with 5% NaHCO₃, 5% KHSO₄ and brine, separated, dried over MgSO₄ and filtered. After removing the solvent, the crude InhA-conjugate 1 was obtained as pale brown solid (264 mg, 0.37 mmol, 74 %) that was used without further purification in the next step.

### 2.9.3 InhA-conjugate 2

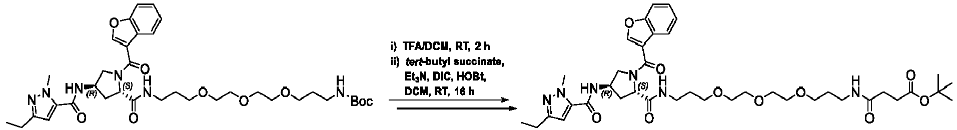

InhA-conjugate 1 (260 mg, 0.36 mmol) was solved in TFA:DCM (1:4, 5 mL) and stirred at room temperature for 2 hours. The solvent was removed under reduced pressure and the crude amine was used in the next step without further purification.
Mono *tert*-butyl succinate (95.4, 0.55 mmol), Et₃N (151.8 µL, 1.09 mmol), DIC (113.1 µL, 0.73 mmol) and HOBt monohydrate (111.7 mg, 0.73 mmol) were solved in DCM (5 mL) and stirred at room temperature. After 15 minutes, the crude amine was added and the resulting mixture was stirred at room temperature for 16 hours.
The reaction was quenched by the addition of 5 % aq. NaHCO₃ (10 mL). The organic phase was separated, dried over MgSO₄ and filtered. After removing the solvent under reduced pressure, the crude InhA-conjugate 2 was used in the next step without further purification.

### 2.9.4 InhA-conjugate 3

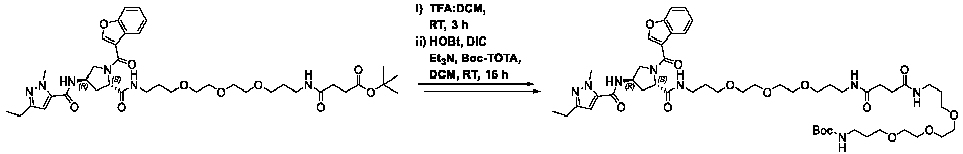

InhA-conjugate 2 was dissolved in DCM:TFA (10 mL, 4:1) and stirred at room temperature for 3 hours. The solvent was removed under reduced pressure and the free acid was redissolved in DCM (10 mL). HOBt monohydrate (111.7 mg, 0.73 mmol), Et₃N (202.4 µL, 1.46 mmol), DIC (113 µL, 0.73 mmol) and Boc-TOTA (175.2 mg, 0.55 mmol) were added. The resulting mixture was stirred at room temperature for 16 hours. The reaction was quenched by the addition of 5 % aq. NaHCO₃ (15 mL). The organic phase was separated, washed with brine (15 mL), dried over MgSO₄ and filtrated. The solvent was removed by evaporation under reduced pressure to obtain the crude InhA-conjugate 3, that was used for the next step without further purification.

### 2.9.5 InhA-conjugate 4

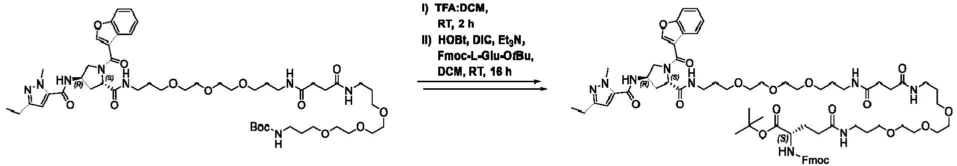

InhA-conjugate 3 was solved in DCM:TFA (5 mL, 4:1) and stirred at room temperature. After two hours, the solvent was removed under reduced pressure.
The obtained residue was redissolved in DCM (10 mL). Fmoc-L-Glu-O*t*Bu (233 mg, 0.548 mmol), HOBt monohydrate (111.7 mg, 0.73 mmol), Et₃N (202.4 µL, 1.46 mmol) and DIC (113 InhµL, 0.73 mmol) were added. The resulting solution was stirred at room temperature for 16 hours.
The reaction was quenched by the addition of 5 % aq. NaHCO₃ (10 mL). The organic phase was separated, washed with brine (10 mL), dried over MgSO₄ and filtrated. the resulting residue was purified by C18-RP-columnchromatography using ACN in water (30 % → 60 %) as eluent system to obtain the desired InhA-conjugate 4 (142 mg, 0.107 mmol, 29 %, 6 steps).

### 2.9.6 InhA-conjugate 5

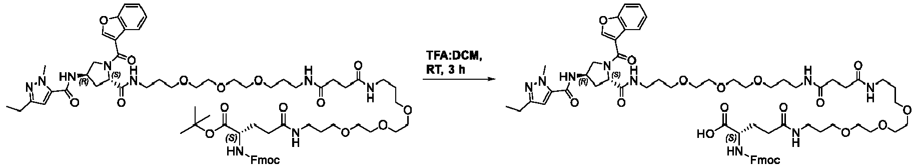

InhA-conjugate 4 (142 mg, 0.107 mmol) was solved in DCM:TFA (4:1, 5 mL) and stirred at room temperature. After 3 hours, the reaction was quenched by the addition of 5 % aq. KHSO₄ (10 mL). The organic phase was washed with brine (10 mL), dried over MgSO₄ and filtrated. The solvent was removed under reduced pressure to obtain InhA-conjugate 5 as a white solid (125 mg, 0.099 mmol, 92 %).

### 2.9.7 Cym_bacPROTAC_09

200 mg 2-chlorotrityl resin (corresponding to 0.32 mmol initial loading) were loaded with 71 mg (0.15 mmol) Fmoc-L-N-Me-Lys(Boc)-OH according to general procedure 1.
The linear peptide was synthesized according to general procedure 2 (using the amino acids Fmoc-L-N-Me-Lys(Boc)-OH, Fmoc-L-Trp(Boc)-OH, Fmoc-L-Ile-OH, Fmoc-N-Me-L-Leu-OH, Fmoc-L-Val-OH, Fmoc-*L*-Phe(3*R*-MeO)-OH and InhA-conjugate 5) and 3, yielding 66 mg (0.032 mmol, 21 %). This peptide was cyclized according to general procedure 4 - method B, yielding 47 mg (0.023 mmol, 72 %) of the cyclized peptide.
This peptide was then deprotected and purified via general procedure 5 - method A to yield 2.8 mg (0.0015 mmol, 7 %) of the desired product.

### Example 3: Determination of dissociation constant (K_{D}) and and Minimal Inhibitory Concentration (MIC)

Binding affinities were determined using isothermal titration calorimetry (ITC).

ITC experiments were carried out using MicroCal PEAQ-ITC (Malvern) at 25 °C, whilst stirring at 750 rpm in a buffer containing 50 mM Tris pH 7.5, 100 mM NaCl. Each titration consisted of 19 injections with intervals of 120 s (the first injection of 0.4 µL followed by 18 injections of 2 µL). DMSO concentration was matched between cell and syringe to 2%. The data were fitted to a single binding site model with a fitted offset subtraction in the MicroCal PEAQ-ITC Analysis Software. Each titration was repeated at least two times.
Titrations were performed with ligand in the syringe and protein in the cell. A control titration of ligand into buffer was performed in order to account for the heat of dilution. Titrations involving compounds with poorer solubility of the compound in buffer were performed with ligand in the cell and protein in the syringe. The following table 2 provides the results of the determination.

**Table 2: results for dissociation constant (K_{D}) and and Minimal Inhibitory Concentration (MIC)**

| Example | compound name | molecular weight (Mw) | K_{D} | MIC |
|---|---|---|---|---|
| 1.1 | CycloA_D2 | 887.09 | 225 µM | n.d. |
| 1.2 | CycloA_D3 | 1043.32 | n.d. | n.d. |
| 1.3 | CylcoA_D4 | 873.11 | 107 µM | > 30 µM |
| 1.4 | CycloA_D5a | 906.18 | 128 µM | > 30 µM |
| 1.5 | CycloA_D5b | 850.07 | 326 µM | n.d. |
| 1.6 | CycloA_D6 | 886.15 | 14.9 µM | 24 µM |
| 1.7 | CycloA_D7 | 928.19 | 26.8 µM | > 120 µM |
| 1.8 | CycloA_D9 | 916.18 | 2.8 µM | 12 µM |
| 1.9 | CycloA_D10 | 954.27 | - | 40 µM |
| 1.10 | CycloA_D11 | 984.30 | - | 20 µM |
| 1.11 | CycloA_D13 | 932.13 | - | >200 µM |
| 1.12 | CycloA_D14 | 872.13 | - | 26 µM |
| 1.13 | CycloA_D15a | 902.11 | - | >200 µM |
| 1.14 | CycloA_D15b | 916.13 | - | 59 µM |
| 1.15 | CycloA_D17 | 996.31 | - | 20 µM |
| 2.1 | Cym_bacPROTAC_01 | 1199.57 | - | - |
| 2.2 | Cym_bacPRQTAC_02 | 1402.81 | - | - |
| 2.3 | Cym_bacPRQTAC_03 | 1559.38 | - | - |
| 2.4 | Cym_bacPROTAC_04 | 1559.38 | - | - |
| 2.5 | Cym_bacPROTAC_05 | 1742.18 | - | - |
| 2.6 | Cym_bacPROTAC_06 | 1492.83 | - | - |

### Biochemical methods:

### DNA constructs:

DNA construct for *E. coli* overexpression of full-length *B. subtilis* ClpC, CIpC_{DWB} (E280A/E618A), ClpC NTD (1-148), ClpP, and BRDT_{BD1} were described in previous publications. *B. subtilis* ClpC, ClpC_{DWB}, ClpC_{NTD} and ClpP carry a C-terminal hexahistidine tag while BRDT_{BD1} has a N-terminal hexahistidine tag followed by a TEV cleavage site.

*M. smegmatis clpP1, clpP2* and *clpC1* genes (MSMEG_4673, MSMEG_4672, MSMEG_6091) were ordered from GeneArt (Thermo Fisher) and cloned into a pET21a vector. ClpP1 and ClpP2 carry a C-terminal tetrahistidine tag, while ClpC1 is untagged. *M. smegmatis clpC1* NTD (1-148) was cloned in pET21a and carries a C-terminal hexahistidine tag.

mSA-fusion constructs with *B. subtilis* proteins (mSA-NrdI, mSA-TagD, mSA-NusA) cloned into a pETM14 vector were purchased from Genewiz. mSA and mSA-Kre constructs were cloned into a pNIC28-Bsa4 vector. These constructs carry a N-terminal hexahistidine tag followed by a TEV cleavage site and a glycine-serine linker between mSA and the bacterial protein. The mSA-Kre construct used for Cryo-EM analysis was cloned into a pET21 vector and has a C-terminal hexahistidine tag (see Table S3 for amino acid sequences of mSA-fusion proteins).

The plasmid for *M. smegmatis* expression of BRDT_{BD1} was ordered from Genescript. It contains the sequence of the BRDT_{BD1} construct in a pMyC vector. pMyC was a gift from Annabel Parret & Matthias Wilmanns (Addgene plasmid # 42192 ; http://n2t.net/addgene:42192 ; RRID:Addgene_42192).

### Protein expression and purification:

Plasmids were transformed into *E. coli* BL21 (DE3) or Rosetta cells (for *M. smegmatis* ClpC1, ClpP1 and ClpP2) and grown in LB broth supplemented with the respective antibiotic at 37 °C. Protein expression was induced by adding 0.1-0.5 mM isopropyl-1-thio-β-D-galactopyranoside (IPTG) once the optical density at 600 nm (OD₆₀₀) had reached 0.8. Cells were cultured overnight at 18-20 °C, harvested the following day by centrifugation and lysed by sonication in a buffer containing 500 mM NaCl, 50 mM Tris pH 7.5, 10 mM Imidazole, and 0.25 mM tris(carboxyethyl)phosphine (TCEP), or flash frozen and stored at -80 °C until purification.

Cell debris was removed by centrifugation. His-tagged proteins were purified from the supernatants using Ni-NTA Agarose beads. After several washing steps, the protein was eluted using 50 mM Tris pH 7.5, 100 mM NaCl, 300 mM imidazole, 0.25 mM TCEP. Eluted protein was loaded onto a size exclusion chromatography column (Superdex 75 16/60 or Superdex 200 16/60 (GE Healthcare) depending on protein size) equilibrated in 50 mM Tris pH 7.5, 100 mM NaCl. For ClpC and ClpC_{DWB} purifications, size exclusion buffer contained instead 300 mM NaCl. Purified fractions were pooled and concentrated before flash freezing and stored at -80 °C.

Cell pellets for ClpP1 and ClpP2 purification were resuspended in 50 mM HEPES-NaOH pH 7.8, 300 mM NaCl, 30 mM imidazole and lysed by sonication. After clarification of the lysate, ClpP1 and ClpP2 were purified by Ni-NTA affinity chromatography (elution buffer: 50 mM HEPES-NaOH, pH 7.8, 300 mM NaCl, 250 mM imidazole) and subsequent gel filtration on a Superose 6 16/60 column (GE Healthcare) equilibrated in 50 mM HEPES-NaOH pH 7.2, 150 mM KCl. 10% glycerol was added to the elution fractions before flash freezing and storage at -80 °C. Processing of the full-length ClpP1 and ClpP2 to the mature ClpP1P2 complex was performed as previously described by Leodolter et al., PLoS One 10, e0125345 (2015).

Cell pellets for ClpC1 purification were resuspended in 50 mM Tris pH 7.5, 75 mM KCl, 2 mM EDTA, 10% glycerol and lysed by sonication. After clarification of the lysate, ClpC1 was precipitated using 40% (w/v) ammonium sulphate. The resulting pellet was resuspended in the original lysis buffer, loaded on a HiLoad 26/10 Q Sepharose High Performance column (GE Healthcare) and eluted with a KCl gradient. ClpC1-containing fractions were pooled and precipitated again using 40% (w/v) ammonium sulphate. The resulting pellet was resuspended in 50 mM HEPES-NaOH pH 7.2, 150 mM KCl, 10% glycerol and loaded on a HiLoad 16/10 Superdex 200 prep grade gel filtration column (GE Healthcare) equilibrated in the same buffer. ClpC1 containing fractions were pooled and stored at -80 °C. Correct molecular mass of the purified proteins was verified by mass spectrometry.

### Example 4: In vitro degradation assays using the compound Cym_bacPROTAC_2

The ClpC1P1P2 protease was reconstituted *in vitro* and used to determine Cym_bacPROTAC_2 induced degradation of mSA.

*In vitro* degradation assays containing 0.5 µM *B. subtilis* ClpC (hexamer), 0.5 µM *B. subtilis* ClpP (heptamer), 2 µM substrate, 15 mM phosphoenolpyruvate (PEP), 10 U/mL pyruvate kinase (Sigma Aldrich) were performed in 50 mM Tris pH 7.5 (at 37 °C), 50 mM KCl, 20 mM MgCl₂, 10% glycerol. The indicated compound concentration (dissolved in DMSO), or only DMSO were added to the reactions, the final concentration of DMSO was 1%. Reactions were started by addition of 5 mM ATP and terminated by adding SDS sample buffer after 2 hours incubation at 37 °C. The samples were resolved by SDS-PAGE and the resulting gels were Coomassie stained. Degradation assays using 0.5 µM *M. smegmatis* ClpC1 (hexamer), 0.25 µM *M. smegmatis* ClpP1P2 (mature 14-mer complex after pro-peptide cleavage), 2 µM substrate, 15 mM PEP, 10 U/mL pyruvate kinase (Sigma Aldrich) were carried out in 50 mM HEPES pH 7.2, 100 mM KCl, 10 mM MgCl₂, 10% glycerol using the same procedure. For each substrate, three independent experiments were performed and showed similar results.

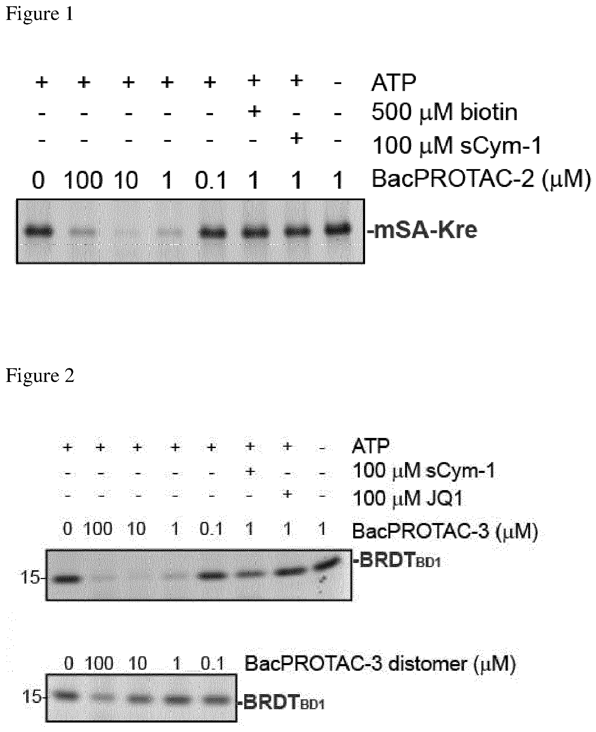

The figure 1 shows the results of the *in vitro* degradation assays using the compound Cym_bacPROTAC_2 under the conditions detailed above. As can be seen in figure 1, the sCymA-based BacPROTACs exhibited a similar efficiency in degrading the mSA model substrate as did the pArg-containing degrader used as a control.

### Example 5: In vivo degradation assay in live mycobacteria using the compound Cym_BacPROTAC_3

The bromodomain-1 (BD1) of BRDT, BRDT_{BD1} (residues 21-137), encodes a small, soluble protein not present in mycobacteria, which binds with high affinity to JQ1, a BET bromodomain inhibitor. The compound Cym_bacPROTAC_3 links sCym-1 to JQ1 and thus targets BRDT_{BD1}.

### 5.1 In vitro degradation assay

In a first step, the compound Cym_bacPROTAC_3 was tested in an *in vitro* assay as described in Example 4. The figure 2 shows the results. As can be seen in figure 2, the compound Cym_bacPROTAC_3 was capable of recruiting BRDT_{BD1} and activate ClpC1P1P2 to induce degradation of BRDT_{BD1} in a stereo-specific manner.

After validating the activity of the compound Cym_bacPROTAC_3 *in vitro,* the compound was investigated in an *in vivo* degradation assay in a cellular environment. For the *in vivo* degradation assay Msm cells stably expressing BRDT_{BD1} were used. The culture was treated with the compound Cym_bacPROTAC_3 or alternatively, the individual building blocks sCym-1 and JQ1. After two hours incubation, BRDT_{BD1} levels were quantified using capillary western as detailed below.

### 5.2 In vivo degradation assay

A stationary culture of *M. smegmatis* mc²-155 carrying a plasmid BRDT_{BD1} expression was diluted 1:200 in 50 mL 7H9 medium supplemented with 50 µg/mL hygromycin. The culture was grown at 37 °C with vigorous shaking to an OD₆₀₀ of ∼1. Expression of BRDT_{BD1} was then induced by addition of 200 µL 50% acetamide in H₂O. After induction, the culture was maintained for 4 hours in the same conditions. The culture was then harvested by centrifugation at 3000 xg for 3 minutes and resuspended to an OD₆₀₀ of 5 in fresh 7H9 medium. 250 µL aliquots of the culture were transferred in wells of a 96-well glass-coated microplate (WebSeal Plate+, Thermo Scientific) and treated with either 1% DMSO, 100 µM, 10 µM, or 1 µM BacPROTAC-3, 100 µM sCym-1, or 100 µM JQ1 (each well with bacterial culture contained 1% DMSO). Each treatment was performed in triplicate. After 2 hours incubation, 200 µL of cell suspension was taken from each well, cells were harvested and the resulting pellets were flash-frozen in liquid nitrogen. Three aliquots of initial cell suspensions were taken before compound addition as a non-treated cells control. The cell pellets were thawed, resuspended in 100 µL TBS (20 mM Tris pH 7.5, 150 mM NaCl) and lysed for 10 minutes using the Bioruptor (Diagenode, 10 cycles, 30 seconds on - 30 seconds off) after adding a small amounts of glass beads to the suspension. The lysates were clarified by centrifugation (15 minutes, 4 °C, 20000 xg), flash-frozen and stored at -80 °C.

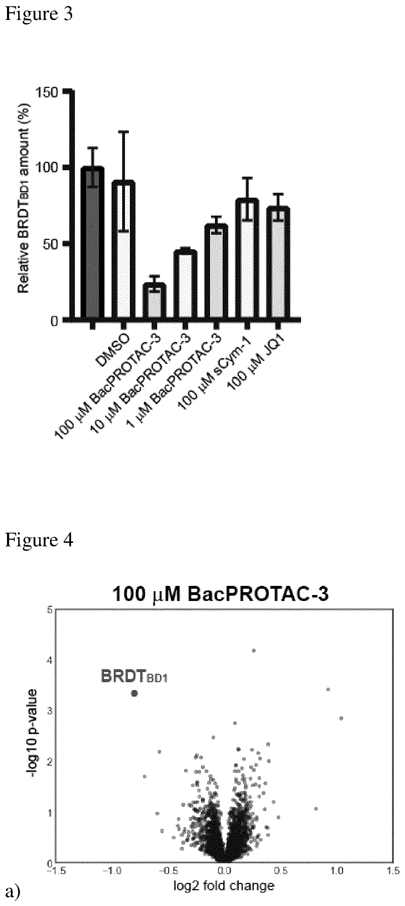

The amounts of BRDT_{BD1} in the lysates were quantified using Wes (ProteinSimple), a capillary electrophoresis-based western blot technology. Bacterial lysates were diluted 2-fold and analysed using the 12-230 kDa Wes Separation Module (Protein simple, SM-W004) following the manufacturer's instructions. Anti-BRDT antibody (Bio-Vision, catalogue # 6643) at 1:200 dilution was used as a primary antibody. BRDT_{BD1} was detected using the Anti-Rabbit Detection Module for Wes (ProteinSimple, DM-001), which includes a peroxidase-conjugated secondary antibody and a chemiluminescent substrate. Results were analysed using the Compass for SW software (ProteinSimple). Compass displays the chemiluminescent signal detected along the length of the capillaries as electropherograms, where the intensity of the chemiluminescent signal is plotted against the apparent molecular weight. Detected peaks were quantified and include a main peak at the expected BRDT_{BD1} molecular weight (BRDT_{BD1} MW = 16.6 kDa) and an additional peak (Peak 2) at high molecular weight (-110 kDa). Peak areas were normalised to non-treated cells control and plotted as mean ± SD, as shown in Figure 3.

Figure 3 illustrates the levels of BRDT_{BD1} expressed in *M. smegmatis* cells after treatment with the compound Cym_bacPROTAC_3 (BacPROTAC-3). The bar chart shows quantification from three independent experiments normalized to BRDT_{BD1} levels before treatment (dark grey bar) and plotted as mean ± SD. As can be taken from figure 3, the compound Cym_bacPROTAC_3 induced BRDT_{BD1} degradation in a concentration dependent manner, while sCym-1 or JQ1 treatments did not affect BRDT_{BD1} levels.

### 5.3 Determination of selectivity of protein degradation

It was assessed whether the compound Cym_bacPROTAC_3 led to a selective elimination of BRDT_{BD1} or had a global impact on the mycobacterial proteome. To address this point, a tandem mass tag (TMT) mass spectrometric (MS) analysis of Msm lysates was performed.

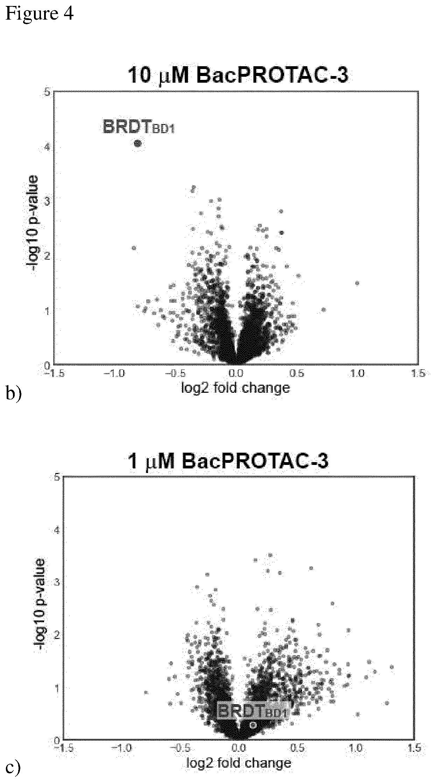

The figure 4 shows the isobaric labelling as volcano plot showing the fold-change (log2) in abundance of 2912 proteins comparing the compound Cym_bacPROTAC_3 (BacPROTAC-3) to vehicle treatment (DMSO), plotted against p-value (-log10) (triplicate analysis). As can be taken from figures 4a), b) and c), isobaric labelling allowed the MS detection and quantification of 2912 proteins, among which only BRDT_{BD1} was significantly (p < 0.001) depleted upon BacPROTAC-3 treatment. In control experiments, it was verified that incubation with the isolated sCym-1 and JQ1 compounds did not affect BRDT_{BD1} levels and did also not cause global effects. Thus, the TMT MS analysis provides compelling evidence that the compound Cym_bacPROTAC_3 is capable of inducing degradation of the BRDT_{BD1} substrate in a highly specific and efficient manner.

The *in vivo* data demonstrate that the compounds of the invention, i.e. the CymA-based BacPROTACs, can hijack ClpC1P1P2 to induce degradation of prteins of interest inside mycobacteria.

## Claims

1. A compound according to general formula (1) or a pharmaceutically acceptable salt, stereoisomer, solvate or polymorph thereof: 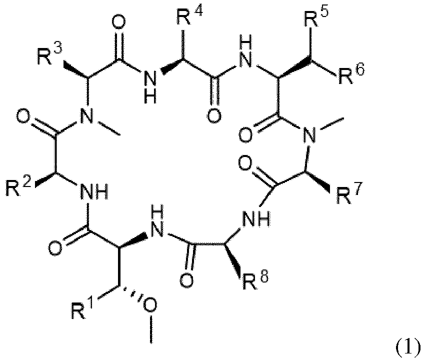 wherein:
R¹ is a 5- or 6-membered aromatic or heteroaromatic ring or a 9- or 10-membered aromatic or heteroaromatic ring system, optionally substituted with one or more groups independently selected from NO₂, CN, OH, halogen, optionally substituted C₁-C₆ alkyl, optionally substituted C₂-C₆ alkenyl, or optionally substituted C₁-C₆ alkoxy where the substitution may be NO₂, CN, OH, COOH, or halogen;
R², R³, R⁴ are each independently selected from the group comprising H, NO₂, CN or halogen optionally substituted C₁-C₆ alkyl, optionally substituted C₂-C₆ alkenyl, optionally substituted C₁-C₆ alkoxy, optionally substituted -(CH₂)ₘ-S-(CH₂)ₘ-CH₃, optionally substituted -(CH₂)ₘ-O-(C₁-C₄) alkyl, optionally substituted -(CH₂)ₘ-OH, optionally substituted -(CH₂)ₘ-CH(OH)-(CH₂)ₘ-CH₃, optionally substituted -(CH₂)ₘ-SH, optionally substituted -(CH₂)ₘ-NR^{1N}R^{2N}, optionally substituted -(CH₂)ₘ-C(O)-NR^{1N}R^{2N}, optionally substituted -(CH₂)ₘ-NH-C(NR^{1N}R^{2N})NR^{1N}, optionally substituted -(CH₂)ₘ-NHC(O)R^{1N}, optionally substituted -(CH₂)ₘ-COOH, where the substitution may be C₁-C₃ alkyl, C₁-C₃ alkoxy, NO₂, CN, OH, COOH, or halogen;
R⁵ is a 5- or 6-membered aromatic or heteroaromatic ring or a 9- or 10-membered aromatic or heteroaromatic ring system, optionally substituted with one or more groups independently selected from NO₂, CN, OH, COOH, halogen, or optionally substituted C₁-C₆ alkyl, optionally substituted C₂-C₆ alkenyl, optionally substituted C₁-C₆ alkoxy where the substitution may be NO₂, CN, OH, COOH, or halogen;
R⁶ is selected from the group comprising H and OH;
R⁷ is -(CH₂)ₙ-NR^{3N}R^{4N};
R⁸ is selected from R² or is a moiety bound to a linker which covalently links the moiety to a targeting group, and the targeting group is capable of binding to a target protein or (poly)peptide which is to be degraded by ClpCP protease;
R^{1N}R^{2N} are each independently selected from the group comprising H and C₁-C₃ alkyl;
R^{3N}, R^{4N} are each independently selected from the group comprising H, OH, COOH, CO, -(CH₂)-OH and C₁-C₃ alkyl;
m is each independently 0, 1, 2, 3, 4, 5 or 6;
n is 1, 2, 3, 4, 5 or 6.

2. The compound according to claim 1, wherein
R¹, R⁵ is independent from each other selected from the group comprising phenyl, phenol, imidazol and indol, optionally substituted with one or more groups selected from C₁-C₆ alkyl, C₂-C₆ alkenyl, C₁-C₆ alkoxy, NO₂, CN, OH, COOH, or halogen.

3. The compound according to claim 1 or 2, wherein the compound is a compound according to general formula (2) or a pharmaceutically acceptable salt, stereoisomer, solvate or polymorph thereof: 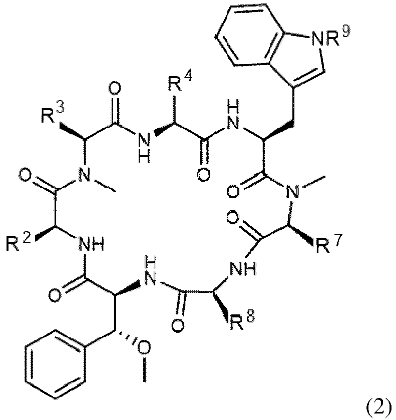 wherein:
R², R³, R⁴ are each independently selected from the group comprising H, optionally substituted C₁-C₆ alkyl, optionally substituted C₂-C₆ alkenyl, optionally substituted C₁-C₆ alkoxy, optionally substituted -(CH₂)ₘ-S-(CH₂)ₘ-CH₃, optionally substituted -(CH₂)ₘ-O-(C₁-C₃) alkyl, wherein the substitution may be NO₂, C₁-C₃ alkoxy CN, OH, or halogen,
R⁷ is -(CH₂)ₙ-NR^{3N}R^{4N};
R⁹ is selected from the group comprising H, C₁-C₆ alkyl, C₂-C₆ alkenyl, or C₁-C₆ alkoxy,
R^{3N}, R^{4N} are each independently selected from the group comprising H, OH, CO,-(CH₂)-OH and C₁-C₃ alkyl;
m is each independently 0, 1, 2 or 3;
n is 1, 2, 3, 4, 5 or 6; and
R⁸ is selected from R² or is a moiety bound to a linker which covalently links the moiety to a targeting group, and the targeting group is capable of binding to a target protein or (poly)peptide which is to be degraded by ClpCP protease.

4. The compound according to any of the preceding claims, wherein
R², R³, R⁴ are each independently selected from the group comprising H, -CH(CH₃)₂,-CH₂-CH(CH₃)₂, -CH(CH₃)CH₂-CH₃ and -(CH₂)₂-S-CH_{3;}
R⁷ is -(CH₂)ₙ-NR^{3N}R^{4N} wherein R^{3N}, R^{4N} are each independently selected from the group comprising H, OH, CO,-(CH₂)-OH and C₁-C₃ alkyl, and n is 1, 2, 3, 4, 5 or 6,
R⁸ is selected from R² or is a moiety bound to a linker which covalently links the moiety to a targeting group, and the targeting group is capable of binding to a target protein or (poly)peptide which is to be degraded by ClpCP protease, and
R⁹ is selected from the group comprising H, C₁-C₆ alkyl, C₂-C₆ alkenyl and C₁-C₆ alkoxy.

5. The compound according to to any of the preceding claims, wherein R⁸ is a moiety bound to a linker which covalently links the moiety to a targeting group, and the targeting group is capable of binding to a target protein or (poly)peptide which is to be degraded by ClpCP protease.

6. The compound according according to claim 5, wherein the linker is selected from polyethylene glycole, a peptide linker, an alkyl linker and/or -((CH₂)ₓN(CH₃))ₙ- wherein x is 1 or 2 and n is an integer in the range of 1 to 24.

7. The compound according to any of the preceding claims, wherein the targeting group is selected from the group comprising biotin, SLF, trimethoprim (TMP) and JQ1.

8. The compound according to any of the preceding claims for use as a medicament, preferably for use in the treatment of mycobacterial infections.

9. A pharmaceutical composition comprising as an active ingredient a compound according to anyone of claims 1 to 7, preferably for use in the treatment of mycobacterial infections.

10. A method of screening for compounds that bind to ClpCP protease, wherein the method comprises the steps of:
- providing a mycobacterial ClpCP protease;
- providing a bacPROTAC compound comprising:
(i) a targeting group for a target protein;
(ii) a linker; and
(iii) a moiety that is a candidate for binding to said ClpCP protease;
- providing a cellular system or a cell lysate system that allows for ClpCP protease directed protein degradation and that comprises said target protein;
- contacting said bacPROTAC compound to be screened with said cellular system or said cell lysate system, to allow complex formation of said bacPROTAC compound with said target protein and said ClpCP protease;
- incubating said cellular system or said cell lysate for an appropriate period of time; and
- determining, after said incubation period, the protein level of said target protein, whereby a reduction of said protein level, compared to a cellular system or cell lysate system to which no bacPROTAC compound has been added, is indicative for ClpCP protease directed protein degradation induced by said bacPROTAC compound, and whereby said moiety (iii) is identified as a compound that binds to said ClpCP protease.

11. The method of claim 10, comprising the steps of:
- providing a mycobacterial ClpC1P1P2 protease comprising a ClpC1_{NTD} receptor domain;
- providing a bacPROTAC compound comprising:
(i) a targeting group for a *M. tuberculosis* target protein;
(ii) a linker; and
(iii) a moiety that is a candidate for binding to said ClpC1_{NTD} receptor domain;
- providing a cellular system or a cell lysate system that allows for ClpCP protease directed protein degradation and comprises said *M. tuberculosis* target protein;
- contacting said bacPROTAC compound to be screened with said cellular system or cell lysate system, to allow complex formation of said bacPROTAC compound with said *M. tuberculosis* target protein and said ClpC1_{NTD} receptor domain;
- incubating said cellular system or cell lysate for an appropriate period of time; and
- determining, after said incubation step, the protein level of said *M. tuberculosis* target protein,
whereby a reduction of said protein level, compared to a cellular system or cell lysate system to which no bacPROTAC compound has been added, is indicative for ClpCP protease directed protein degradation induced by said bacPROTAC compound, and whereby said moiety (iii) is a candidate for binding to said ClpC1_{NTD} receptor domain.

12. A method of screening for target proteins for induced protein degradation, wherein the method comprises the steps of:
- providing a compound according to anyone of claims 1 to 7 comprising a ClpC1_{NTD}-binding synthetic cyclic heptapeptide and a targeting group for a carrier protein or protein domain;
- providing modified mycobacterial cells that express a *M. tuberculosis* target protein, such as InhA or ThyA, which is coupled to said carrier protein or protein domain;
- incubating said modified mycobacterial cells with said compound; and
- analyzing the vitality of the modified mycobacterial cells,
whereby a reduction of living cells is indicative of a target protein for induced protein degradation of the target protein, and whereby such target protein is identified as a suitable target for bacPROTAC compound induced protein degradation.

13. A method of screening for a targeting group for a *M. tuberculosis* target protein, wherein the method comprises the steps of:
- providing a library of bacPROTAC compounds according to anyone of claims 1 to 7 comprising a ClpC1_{NTD}-binding synthetic cyclic heptapeptide and linked to a potential targeting group for a *M. tuberculosis* target protein;
- providing *M. tuberculosis* mycobacterial cells;
- incubating said *M. tuberculosis* mycobacterial cells with a compound of said library of bacPROTAC compounds; and
- determining induced degradation of the *M. tuberculosis* target protein by determining vitality of said mycobacterial cells compared to *M. tuberculosis* mycobacterial cells which were not incubated with a compound of said library of bacPROTAC compounds,
whereby a reduction of said vitality of said cells upon incubation with said bacPROTAC compound is indicative of a bacPROTAC compound that includes a targeting group for a *M. tuberculosis* target protein.

14. Use of a compound according to anyone of claims 1 to 7 for the manufacture of a medicament, preferably for the manufacture of a medicament for the treatment of mycobacterial infections, particularly of tuberculosis or atypical mycobacterial infections.

15. A method of treating mycobacterial infections, particularly of tuberculosis, the method comprising the step of administering to a subject a therapeutically effective amount of a compound according to anyone of claims 1 to 7.
